# EUROPEAN PATENT SPECIFICATION

(11) **EP 0 828 459 B1**
(45) Date of publication and mention of the grant of the patent: **24.09.2003**
(21) Application number: 96912660.6
(22) Date of filing: 09.04.1996
(51) Int. Cl.: A61B 17/70, A61B 17/80

(54) **A POLYAXIAL LOCKING SCREW COLLAR AND PLATE ASSEMBLY**
MEHRACHSIGE VERRIEGLUNGSANORDNUNG BESTEHEND AUS EINER SCHRAUBE, EINER SCHELLE UND EINER PLATTE
ENSEMBLE POLYAXIAL DE VERROUILLAGE COMPRENANT VIS, COLLIER ET PLAQUE

(30) Priority: 13.04.1995 US 421087
(43) Date of publication of application: 18.03.1998
(60) Divisional application: 03015953.7
(73) Proprietor: Fastenetix, L.L.C., Summit, NJ 07901 (US)
(72) Inventor: ERRICO, Joseph, P., Far Hills, NJ 07931 (US); ERRICO, Thomas, J., Summit, NJ 07901 (US); RALPH, James, D., Oakland, NJ 07436 (US); TATAR, Steve, Montville, NJ 07045 (US)
(74) Representative: Gates, Marie Christina Esther
(86) International application number: PCT/US96/04920
(87) International publication number: WO 96/032071

(56) References cited:
- WO-A-88/03781
- US-A- 4 493 317
- US-A- 5 057 111

## Description

### Background of the Invention:

### 1. Field of the Invention:

This invention relates generally to a orthopaedic implant assemblies for holding adjacent bones fixed. More particularly, this invention relates to a novel assembly of bone screws, collars, and plates for use in surgical procedures for stabilizing the relative motion of, or permanently immobilizing, bones, wherein the screws and collars form polyaxial couplings which are lockable in a receiving hole in a plate through a wide range of angulations.

### 2. Description of the Prior Art:

Human bones are constructed of a hard external shell, referred to as cortical bone, and an interior which is less dense and is referred to as cancellous bone. Although bones are generally resilient and structurally remarkable with respect to normal long term cyclical loading, extreme loading, a variety of traumas, and diseases may cause bones to fracture. Long bone fractures may be classified according to their severity, ranging from minor stress fractures to fully displaced fractures.

In cases of serious fractures of long bones, including those in which bone continuity is totally disrupted by a complete displacement of segments thereof, commensurately serious measures must be applied to correct the damage, and to permit a "callus" to form around the bone whereby union of the fracture is achieved. In many cases it is sufficient to "set" the bone by initially placing the surfaces of the bone segments which need to join in contact with one another, and applying rigid external immobilization. External casts and braces are utilized to provide stabilization of the bone, as well as to eliminate excessive stress loading during the healing process. Stress loadings of a light to moderate level are conducive to bone growth, however, if the stresses during healing are excessive, the fracture may not heal, and will alternatively go on to form a "non-union".

In the most severe of instances, however, the segments of the bone which is broken, and the manner in which the break manifests itself, makes the reduction of the fracture and the maintenance of the reduction difficult. In some cases the bone segments are displaced to the extent that invasive surgical procedures must be carried out in order to openly reduce the bone segments together. In others, the specific bone which is broken (such as the femur or the hip) is one for which the time involved with total healing exceeds what may practically be externally immobilized. In still others, the ability of a cast to sufficiently relieve the loadings associated with prudently constrained activity is insufficient to permit correct healing. In such cases it is often necessary to openly reduce and internally affix metal devices such as screws, rods, and plates to the bone segments

Such a screw plate assembly is known from WO88/03781 which relates to a device in which bone screws are connected in a rigid and stable manner with an osteosynthesis plate. The screws are inserted through bone holes in the plate into the bone and fixed in place with an adjusting screw.

An important class of orthopaedic conditions for which immobilization via internal fixation is frequently required concerns the spine. The spinal column of bones is highly complex in that it includes over twenty bones coupled to one another, housing and protecting critical elements of the nervous system having innumerable peripheral nerves and circulatory bodies in close proximity. In spite of these complications, the spine is a highly flexible structure, capable of a high degree of curvature and twist in nearly every direction.

Genetic or developmental irregularities, trauma, chronic stress, tumours, and disease are a few of the causes which can result in spinal pathologies for which permanent immobilization of multiple vertebrae may be necessary. A variety of systems have been disclosed in the art which achieve this immobilization by implanting artificial assemblies in or on the spinal column.

The region of the back which needs to be immobilized, as well as the individual variations in anatomy, determine the appropriate surgical protocol and implantation assembly. The use of screw and plate assemblies for stabilization and immobilization via lateral or anterior entrance is common. Additionally, the use of plate assemblies in the posterior cervical spine is also common.

Because bones, once healed (or, in the case of immobilized sequences of vertebrae, fused) are routinely subject to high loads which cycle during movement, one of the primary concerns of physicians performing implantation surgeries, as well as of the patients in whom the implants are placed, is the risk of screw pull-out. Screw pull-out occurs when the cylindrical portion of the bone which surrounds the inserted screw fails. A bone screw which is implanted perpendicular to the plate is particularly weak because the region of the bone which must fail for pull-out to occur is only as large as the outer diameter of the screw threads. It has been found that for pull-out to occur for a pair of screws which are angled inward, "toe nailed", or ones which diverge within the bone, the amount of bone which must fail increases substantially as compared with pairs of screws which are implanted in parallel along the axis that the loading force is applied. It has, therefore, been a goal of those in the art to provide screw plate assemblies which permit screws to be entered into the bone at angles other than 90 degrees.

Another concern, especially in the spine, is that there are important internal tissue structures which, because of their proximity to the implant, may be damaged by a dislocated screw. In the cervical spine for example, the esophagus is located directly in front of the anterior surface of the vertebral body, and therefore, in potential contact with an implanted anterior cervical plate. Breaches of the esophageal wall permit bacterial contamination of the surrounding tissues, including the critical nerves in and around the spinal cord. Such contamination can be fatal.

Because screw pull-out represents one of the largest risks of implant failure, it has been an object of those in the art to produce a screw and plate designs having a locking means which couples, not only the plate to the bone, but locks the screw to the plate. In such a design, it is intended that, even if the bone holding the screw fails, the screw will not separate from the plate.

With respect to the reduction of long bone fractures, a very successful advance over standard screw plate assemblies are dynamic compression plates (DCPs). In its most widely used form, the DCP is an elongate planar metal plate having a plurality of sequentially spaced elongated holes, the holes being coaxial and extending along the axis of the plate. Each hole, which extends fully through the plate, is defined by a pair of vertically oriented parallel sidewalls. The top of the parallel sidewalls, near the top surface of the plate, includes an additional recessed lip on which the annularly extending portion of the head of a screw rests once inserted into the hole. The top of the lip is, therefore, lower than the top surface of the plate. The elongate profile of the lip defines a downward slope with respect to the top surface of the plate, wherein the distance between the top of the lip surface and the top surface of the plate increases along the elongate axis of the hole. The downward slope of the lip of each hole is oriented toward the middle of the plate.

In use, the plate is positioned next to the exposed surface of the broken, and potentially displaced, bone such that one portion, an upper portion of the plate is adjacent to one of the segments, and the holes of the lower portion, are adjacent to the other bone segment. First and second screws are inserted through the plate into opposing segments of the broken bone, at respective points along the axial extent of the elongate holes. It is preferred that the respective insertion points be at least some distance from the end of the hole having the lip at its greatest depth, herein referred to as the proximal end. In general, the screws are generally inserted at the uppermost end of the elongate holes, herein referred to as the remote end.

Once inserted, the screws are driven into the bone, therein securing the plate to the respective bone segments. The compressing function of the plate is utilized by continued driving of the screw into the plate once the annularly extending portion of the screw head has seated against the recessed lip of the hole. The downward force applied to the screw causes the head to slide down the slope of the recessed lip, thereby causing the bone in which it is inserted to move relative to the fixed plate. As the slopes of the respective holes are mutually directed toward the middle of the plate, the relative motion of the screws down the sloped lips, and the corresponding motion of the bone segments into which they have been inserted, is together. The surgeon is thereby able to draw displaced bones together, and to hold the bones together internally during the required healing time.

Unfortunately, however, dynamic compression plates suffer the same failure mechanisms as other screw and plate devices, e.g., screw pull-out. While variations of the DCP concept, specifically those which include additional holding screws for anchoring the plate once compression has been applied, have reduced the dangers associated with bone screw pull-out with such plates, the risks associated with this failure mechanism have not been eliminated. Other DCP systems which have provided for angulation of screws have generally been limited to single angles. Surgeons ideally need to have freedom with respect to insertion angles so that the individual variations in fracture type, and bone anatomy may be accounted for in the fixation means. At the same time, however, once implanted, the screws should not be subject to pull-out.

The present invention is, therefore, directed to providing a new and novel screw and plate design having a polyaxial coupling of the screw to the plate, whereby a single plate is compatible with a wide range of screw-in angles.

Further, the present invention provides a simple and effective locking mechanism for locking the bone screw to the plate.

It is additionally a principal feature of embodiments of the present invention to provide a new and novel dynamic compression plate having a polyaxial coupling of the screw to the plate.

Another principal feature of aspects of the present invention is the ability to provide a dynamic compression screw plate having a locking compression screw and hole.

Other features and advances of the present invention not explicitly stated will be set forth and will be more clearly understood in conjunction with the descriptions of the preferred embodiments disclosed hereafter.

### Summary of the Invention:

The preceding objects of the invention are achieved by the present invention as claimed in claim 1 which is a locking polyaxial screw plate assembly for use in stabilizing and immobilizing bones. In its various embodiments, the invention comprises a plate having a plurality of holes, bone screws having semi-spherical heads, and coupling elements. The coupling elements may comprise a variety of different embodiments, however, each includes an interior volume shaped to receive and polyaxially retain the semi-spherical head therein, and an exterior conformation for insertion and locking into the holes of the plate. The coupling elements can each include a slot and/or circumferential discontinuity such that the radius of the element may be expand or contract by the application of a radial force whereby the interior conformation may compress against the semi-spherical head top portion of the bone screw once the coupling element is inserted and locked in the hole, thereby locking the screw in the desired angle.

In its first embodiment, which is an anterior spinal implant having a unitary coupling element, the plate is a flat metal element, having rounded corners, contoured to the curved cylindrical surface of the vertebral bodies to which it is secured. There are four tapered holes disposed in pairs at the opposing ends of the plate. These holes may be threaded or smooth (corresponding to the type of coupling element being utilized in conjunction therewith), and are positioned so that they are aligned in pairs with the vertebral bodies to which the plate is to be attached.

The threading and shaft portion of the bone screws may be of a variety of standard designs, or a particular design which may be found more secure than the standard ones. The head, however, is not standard in that it comprises a semi-spherical section. For the purposes of inserting the screw into the bone, the head comprises a recessed region such as a slot, phillips, star, or hexagonal recesses which are ideally suited for mating to an appropriate screwdriving tool. The recess, however, does not alter the exterior radially symmetric, semi-spherical shape, of the head.

The coupling element comprises an interior volume which holds the ball head of the screw, and an exterior surface taper which conforms to the taper of the corresponding hole. The coupling element may be threaded or smooth, but in either instance the coupling element includes at least one vertical slot such that the coupling element may expand and contract to receive and/or lock the head of the screw in the interior volume. The size of the tapered hole, relative to the coupling element, is such that the full insertion of the element into the hole causes the slots to contract and the interior surface of the coupling element to lock to the surface of the screw head.

In the variation of this anterior spinal plate in which the coupling element and the tapered holes are smooth, it is understood that the final step of inserting the screw into the bone causes the coupling element to seat in the hole and to be drawn down into the hole, thereby locking the coupling element to the screw, the screw at a desired angle relative to the plate, and the coupling element within the hole. In the variation of this embodiment, suggested above, in which the coupling element and the tapered holes include corresponding threadings, it is understood that the final step of the insertion of the screw and coupling element into the holes further includes a rotation of the coupling element into the hole. This is naturally occurring in a device wherein the threading of the collar is of the same helical orientation as the screw shaft. In the alternative, a separate screwdriving tool may be utilized to insert the coupling element into the plate. In this case, as above, the act of locking the coupling element in the hole causes the interior surface of the coupling element to crush lock to the surface of the head of the screw, thereby locking the screw at its proper angulation relative to the plate. Each of these variations may further include retaining rings integrally formed within the upper portions of the holes. Each retaining ring expands as the corresponding coupling element is inserted into the hole, and snaps back into a retaining position above the coupling element once the top of the element passes it into the hole.

In both variations, the top of the coupling element provides an opening which may be positioned co-linearly with the recess in the screw head for driving in the screw. This opening is desirably large enough such that the screw and coupling element may be manipulated easily and the head of the screw is accessible even at entrance angles which are far from perpendicular.

The first step in the process of implanting these types of anterior spinal implants is to position the plate against the vertebral bodies and to align the entry points for the screws. The next step is to pre-drill the holes into the bones at the desired angle, into which the screws will be inserted. With the plate in place, the screws may now be inserted through the holes in the vertebral bodies. Once the screw has been substantially fully inserted into the bone, at the desired angle, the coupling element contacts the top of hole in the plate. If the coupling element is not aligned properly with the hole, it rotates via its polyaxial mounting to the screw to seat into the hole so that it may be locked down into the plate (either by continued driving of the screw into the bone, or, in the case of the threaded coupling element, by separate rotation of the coupling element).

An alternative embodiment for use in the anterior spine, includes a similar plate having a set of holes for receiving a screw and coupling element therethrough. In this embodiment, however, the holes are untapered and threaded through the top portion thereof, and tapered and smooth at the bottom. In addition, the coupling elements are not unitary elements, but are rather two-piece elements. The top piece of the coupling element has an external threading for locking with the threaded end of the holes in the plate, and also has an interlocking mechanism for joining with the lower piece of the coupling element. The lower piece of the coupling element has a semi-spherical interior volume, which is expandable and contractable, for receiving the semi-spherical head of the screw therein. The bottom portion of the lower piece of the coupling element further includes an exterior taper which mates with the tapered bottom of the corresponding hole in the plate, so that there is an inwardly directed force applied to the lower piece, thereby causing the interior volume of the coupling element to contract and lock to the head of the screw.

More particularly, in this two-piece coupling element embodiment, the two part coupling element comprises and socket portion and a cap portion. The socket portion is designed with an interior semi-spherical volume, so that it may receive the semi-spherical head of a corresponding bone screw. The interior volume of the socket portion is open at both axial ends thereof. The exterior surface of the socket portion, at the bottom thereof, includes a first set of slots which extend upwardly from the opening so that the interior semi-spherical volume may be expanded or contracted by the application of a radial force. In addition, the exterior surface at the bottom is tapered so that it is narrower at the bottom than at a midpoint.

The upper exterior surface of the socket portion comprises a second set of slots, directed axially along the element to the midpoint, such that the upper opening of the socket element may expand and contract in accordance with the application of a radial force thereon. The exterior surface of this upper section of the socket portion is not tapered and is narrower than the widest taper position of the bottom of the socket portion. The upper section, however, does further include an outwardly extending annular lip at the uppermost axial position. This upper section is designed to be inserted into, and joined with, the cap portion of the coupling element.

The cap portion has a generally cylindrical shape, having an open bottom. The open bottom is inwardly tapered, forming an inwardly extending annular lip, so that as the upper end of the socket portion is inserted, its upper slots are narrowed. Once axially inserted beyond this taper, the upper section of the socket portion expands outward over the inwardly extending annular lip. The inwardly extending annular lip engages the outwardly extending lip of the socket portion so as to prevent disengagement of the two pieces. The socket portion is then permitted to slide into the cap portion, until the larger diameter of the tapered lower portion of the socket contacts the entrance of the cap portion.

The exterior surface of the cap portion is threaded, so that it may engage the threading of the upper portion of the corresponding hole. In addition, the top of the cap includes an opening so that a screw driving tool may directly engage the top of the screw.

The first step in the process of implanting this embodiment of the invention is to assemble the parts described above. (It is intended that this assembly occur at the manufacturing site, and not be the responsibility of the surgical staff.) The semi-spherical head of the screw is inserted into the interior volume of the socket portion, and held in place by the interference fit of the maximum diameter of the head with the unexpanded openings at the top and bottom of the element. The head is inserted from the bottom, and more specifically by applying a pressure which causes the bottom opening to expand to receive the head into the volume.

Once the screw head is inserted, the upper end of the socket portion is inserted into the opening of the cap portion. This insertion causes the top of the socket portion to contract inward slightly until the annular lips of each portion engage one another. The socket portion and the cap portion are thereby joined loosely so that each may slide and rotate relative to one another.

The surgeon then positions the plate against the vertebral bodies and aligns the entry points for the screws. The next step is to pre-drill the holes into the bones at the desired angle, into which the screws will be inserted. With the plate in place, the screws (with the corresponding socket and cap portions in place on the head of each screw) are inserted through the holes of the plate, and into the vertebral bodies. The coupling element provides axial access to the screw head for driving it into the bone. At all times during this insertion, the head of the screw is loosely retained within the coupling element, so that the coupling element may polyaxially angulate relative to the screw, within a range of angles defined by the diameter of the neck of the screw and the bottom opening of the socket portion.

As the screw is inserted into the bone, at the desired angle, the socket portion of the coupling element angulates so that the tapered bottom portion thereof seats into the tapered bottom of the hole in the plate. Continued driving of the screw into the bone, and therefore the socket portion of the coupling element deeper into the tapered hole, causes the first set of slots in the bottom end of the socket portion to narrow, thus causing the head of the screw to be crush locked to the coupling element. The cap portion of the coupling element is then threadably inserted into the hole, locking the coupling element in the hole, and further driving the socket portion into the hole.

In a preferred variation of this embodiment, the interior surface of the cap portion includes a slight narrowing taper so that as the cap is threaded downward into the hole in the plate, the upper slots of the socket portion are also narrowed, further increasing the crush locking effect on the head of the screw.

It shall be understood that the shape of the plate, and the distribution of the holes thereon may be altered to correspond to other areas of the spine, for example the posterior cervical region wherein the plate may be aligned against and coupled to the lateral masses. Further, as a standard bone plate, the polyaxial locking screw/coupling element/plate features described in the preceeding embodiments may be applied as general bone plates.

An advanced form of such a general bone plate embodiment, however, requires additional description inasmuch as it provides dynamic compression. More particularly, the dynamic compression plate aspect of the present invention has several alternative embodiments, two of which are introduced hereinbelow. Each embodiment includes an elongate flat metal plate element which is contoured to the curved cylindrical surface of the specific bone to which it is to be secured. In addition, it is desirable that the plate also comprise a plurality of round holes for receiving therethrough additional holding screws which may be polyaxial screws and coupling elements as are set forth above.

In each dynamic compression embodiment, the coupling element of this embodiment comprises a socket for holding the ball head of the screw, and an external conformation which permits the screw and coupling element to lockably mate with the sidewall morphology of the corresponding elongate hole.

More specifically with respect to the sidewall morphology, in a first embodiment, the at least one elongate hole (preferably a pair of holes) extends in the direction of the elongate axis of the plate. The sidewalls of the elongate hole of this embodiment are tapered in two axes; the first axis being perpendicular to the plane of the plate, such that the hole is narrower at the bottom than at the top, and the second axis being coaxial with the hole itself, such that the hole is wider at the end closest to the middle of the plate (as measured with respect to the longitudinal axis of the plate), the proximal end.

The coupling element which corresponds to this first embodiment, therefore, comprises a cylindrical element, tapered to form a frusto-conical section. It is effectively identical to the coupling element of the first embodiment of the anterior spinal screw plate assembly introduced above (with the smooth exterior surface). The width of the top of the elongate hole, at the remote end thereof which is farthest from the middle of the plate, the remote end, is slightly wider than the diameter of the bottom of the coupling element, and is considerably narrower than the top of the coupling element. The tapered sidewalls of the plate widen in the direction of the middle of the plate, toward the proximal end wherein the width of the top of the elongate hole at the proximal end, is equal to the diameter of the top of the coupling element. The width at the bottom of the elongate hole widens as well, in the direction of the middle of the plate, however, at the proximal end, the width of the bottom of the hole widens at a slower rate than the top of the hole. It is this slight difference in tapering which compression locks the coupling element to the screw head and to the plate.

The first step in the process of implanting this embodiment of the invention is to position the plate against the segments of the broken bone which require compression and immobilization, and to align the entry points for the screws. The next step is to pre-drill holes into the bones at the desired position along the elongate hole, and angle with respect to the plate, into which the screws will be inserted. With the plate in position, the first and second screws are inserted through the elongate holes into the bone segments.

Once the screw has been inserted into the bone, at the desired angle, the coupling element, via its rotationally free mating to the socket in the inserted screw, is realigned so that it seats in the elongated hole, the tapered surface of the coupling element fitted to the tapered walls of the hole. Continued rotation of the screw causes the coupling element to slide deeper along the walls of the hole, which correspondingly causes the screw and bone to move relative to the plate. The coupling element continues to slide down the ever widening tapered walls of the hole, until it reaches the proximal end. At the proximal end, the slightly narrowed bottom of the hole crush locks the coupling element to the screw head, therein locking the screw to the coupling element, as well as compression locking the coupling element to the plate.

In the second embodiment of the dynamic compression plate, the elongate holes are not tapered along the axis which is perpendicular to the plane of the plate, but rather each sidewall comprises a sloped channel. This elongate hole does, however, retain a slight taper at the proximal end of the hole. At the remote end, the channel comprises only a recessed lower lip, the lip forming the upper extent of the lateral channel which is sloped downward into the plate. Once the channel has extended a distance large enough to provide an initial seating of the coupling element onto the lower lip, an upper lip portion, which defines the upper extent of the channel, is introduced.

The coupling element of this embodiment comprises an annular flange which seats on the lower lip of the hole in an initial position, and is driven by continued insertion of the screw down the slope of the channel. Once the coupling element begins to translate down the channel, the upper lip extends over the annular flange and prevents the coupling element from pulling out. As above, this coupling element includes at least one vertical slot, either extending downwardly from the top (such that the element compresses onto the head of the screw from above), upwardly from the bottom (such that the element compresses onto the head of the screw from below), or completely from the top to the bottom of the device (a single slot thereby rendering the coupling element as a 7/8ths ring, and such that the element compresses onto the head entirely circumferentially).

Implantation of this apparatus is initiated in a manner which is generally similar to the first embodiment: the plate and holes are aligned, the pre-drill holes are provided at the appropriate angulations, the screws and their corresponding coupling elements are driven through the elongate holes and into the bone. Once the screw is inserted, the annular flange of the coupling element seats on the lower lip of the channel and begins to translate down the slope in accordance with continued driving of the screw into the bone. The bone and screw move relative to the plate, both moving closer to the middle of the plate. As the annular flange of the coupling element travels, it becomes vertically locked beneath the upper lip of the channel. The tapering of the hole in the axial direction, at the proximal end, causes a radial compression of the coupling element, therein locking the ball head to the coupling element, in the same manner as the first embodiment (i.e., slots in the coupling element), but locking the screw to the plate via the annular flange and the channel.

### Brief Description of the Drawings:

Figures 1a and 1b are perspective views of locking plates which are aspects of the embodiments of the present invention.
Figure 2 is a perspective view of a bone screw which is an element of one aspect of the present invention.
Figures 3a and 3b are side views of coupling elements which are aspects of embodiments of the present invention.
Figures 4a and 4b are side cross-sectional views of the fully assembled embodiments of the present invention, the elements of which are shown in Figures 1a, 2, and 3a, and Figures 1b, 2, and 3b, respectively.
Figure 5 is a top view of an alternative plate of the present invention further including integral retaining rings formed in the upper portion of the holes.
Figure 6 is a side cross-section view of the fully assembled form of the embodiment of the present invention including the plate as shown in Figure 5.
Figure 7 is a side cross sectional view of a hole in the plate of another embodiment of the present invention.
Figures 8a and 8b are side cross sectional views of cap and socket portions of a two-piece coupling element of another embodiment of the present invention.
Figure 9 is a side cross section view of a fully assembled form of the embodiment of the present invention which includes the elements illustrated in Figures 2, 7, and 8a-b.
Figure 10 is a side cross section view of a fully assembled form of a variation of the embodiment of the present invention shown in Figure 9, wherein the inner surface of the cap portion is tapered.
Figure 11 is a side view of a dynamic compression plate of the present invention.
Figure 12 is an axial side view of an an elongate hole of an embodiment of the dynamic compression plate aspect of the present invention.
Figure 13 is a side cross-section view of an assembled dynamic compression plate which includes the elements shown in Figures 2, 3a, 11 and 12.
Figure 14 is a side view of a coupling element for use with an alternative dynamic compression plate.
Figures 15a and 15b are side cross-section views, taken along mutually perpendicular axes, of an elongate hole of another embodiment of a dynamic compression plate of the present invention.
Figure 16 is a side cross-section view of the fully assembled dynamic compression plate embodiment, the elements of which are shown in Figures 2, 14, and 15a-b.

### Detailed Description of the Preferred Embodiment:

While the present invention will be described more fully hereinafter with reference to the accompanying drawings, in which particular embodiments and methods of implantation are shown, it is to be understood at the outset that persons skilled in the art may modify the invention herein described while achieving the functions and results of this invention. Accordingly, the descriptions which follow are to be understood as illustrative and exemplary of specific structures, aspects and features within the broad scope of the present invention and not as limiting of such broad scope. Like numbers refer to similar features of like elements throughout.

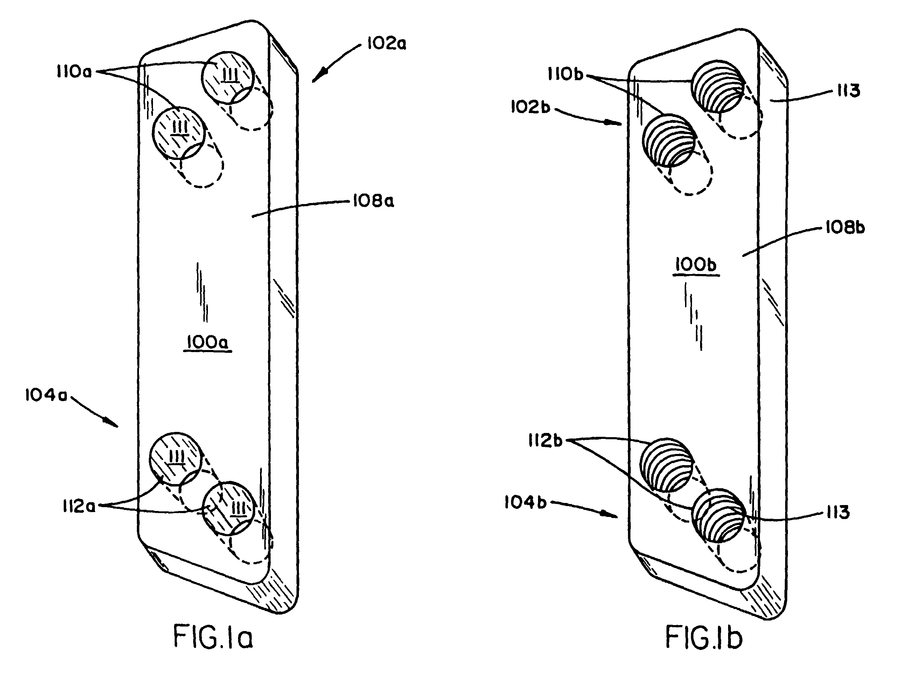

Referring now to Figure 1a, a plate which is an element of the present invention is shown in a perspective view. The plate 100a may be constructed of any suitably biocompatible material which has the structural strength and durability to withstand the cyclical loading associated with long term fixation to the spine. Materials which would be suitable for such applications include titanium alloys and steels. A specific titanium material which has been utilized in implants of the prior art include ASTM F-136 titanium alloy (Ti 6AL-4V). This material has enhanced mechanical properties including fatigue endurance and tensile strength, as compared with pure titanium.

The plate 100a comprises upper and lower portions 102a,104a respectively, and a top surface 108a and a bottom surface (not shown). A slight curvature is generally imparted to the plate 100a so that it may grossly conform to the cylindrical morphology of the bones (i.e., vertebral bodies) which it couples. The curvature is correspondingly such that the top surface 108a is the convex surface, the bottom surface is concave.

A pair of holes 110a, having a smooth tapered inner surface 111, extend fully through the upper portion 102a of the plate. A second pair of holes 112a having a tapered inner surface 111 as well, are disposed in the lower portion 104a of the plate 100a. Each of the holes 110a,112a is ideally suited for receiving therethrough a bone screw for affixing the plate to the vertebral bodies.

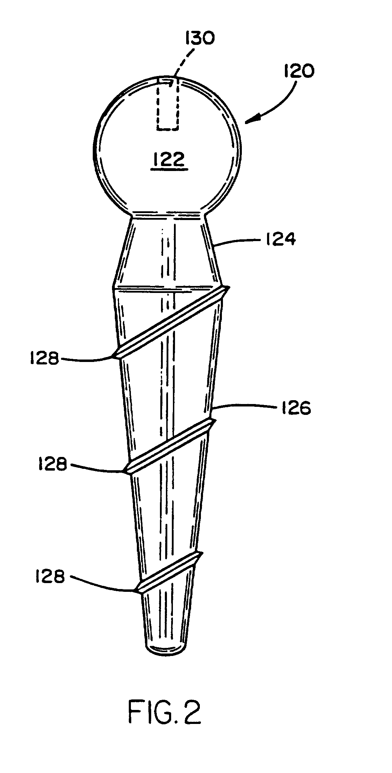

Referring now to Figure 2, a screw of a type which is ideally suited for coupling the plates of this invention to vertebral bodies (or long bones in other embodiments) is shown in a side view. The screw 120 comprises a head portion 122, a neck 124, and a shaft 126. In Figure 2, the shaft 126 is shown as having a tapered shape with a high pitch thread 128. It shall be understood that a variety of shaft designs are interchangeable with the present design. The specific choice of shaft features, such as thread pitch, or shaft diameter to thread diameter ratio, or overall shaft shape, etc. should be made be the physician with respect to the conditions of the patient's bone, however, this invention is compatible with a wide variety of shaft designs.

The head portion 122 of the screw 120 comprises a semi-spherical shape, which has a recess 130 in it. It is understood that the semi-spherical shape is necessarily is a section of a sphere, greater in extent than a hemisphere, and exhibits an external contour which is equidistant from a center point of the head. In a preferred embodiment, the major cross-section of the semi-spherical head 122 (as shown in the two dimensional illustration of Figure 2) includes at least 270 degrees of a circle.

The recess 130 defines a receiving locus for the application of a torque for driving the screw 120 into the bone. The specific shape of the recess 122 may be chosen to cooperate with any suitable screwdriving tool. For example, the recess 130 may comprise a slot for a flat-headed screwdriver, a crossed recess for a phillips head screwdriver, a hexagonally shaped hole for receiving an alien wrench, or most preferably, a threading for receiving a correspondingly threaded post. It is further preferable that the recess 130 be co-axial with the general elongate axis of the screw 120, and most particularly with respect to the shaft 126. Having the axes of the recess 130 and the shaft 126 colinear facilitates the step of inserting the screw 120 into the bone.

The semi-spherical head portion 122 is connected to the shaft 126 at a neck portion 124. While it is preferable that the diameter of the shaft 126 be less than the radius of the semi-spherical head 122, it is also preferable that the neck 124 of the screw 120 be narrower than the widest portion of the shaft 126. This preferable dimension permits the screw to be inserted at a variety of angles while still permitting the specific coupling element to be screwed into the appropriate hole 110a or 112a of the plate 100a and remain coupled to the head 122.

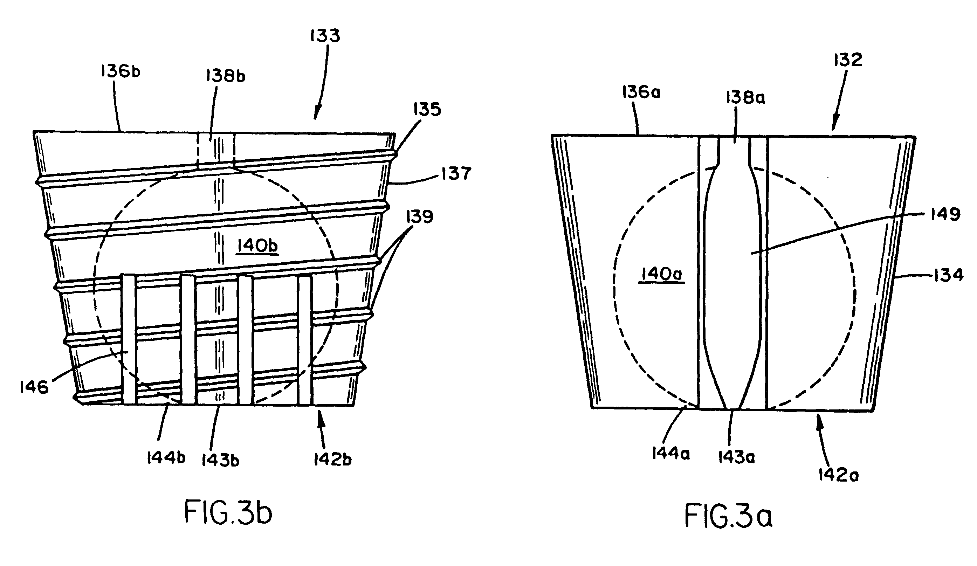

Referring now also to Figure 3a the first variation of the coupling element 132, which is utilized in conjunction with the plate 100a shown in Figure 1a, is shown in side views, wherein phantom lines show the interior structure of the elements along a diametrical cross section. The coupling element 132 comprises a tapered cylindrical socket having a smooth external surface 134. The taper of the surface 134 is designed to mate with the interior tapered surface of the holes 110a or 112a of the plate 100a, so that the coupling element 132 may be seated into the plate 100a.

The top surface 136a of the coupling element 132 further comprises a through hole 138a, which extends from the top surface 136a to an interior semi-spherical volume 140a. This through hole 138a is designed such that the screwdriving tool which is used to insert the screw 120 into the bone may access and rotate the screw 120 through the coupling element.

The coupling element 132 further includes an interior semi-spherical volume 140a which is ideally suited for holding the head portion 122 of the screw 120, and permitting the screw to rotate through a range of angles. The bottom 142a of the coupling element 132 has a circular hole (enumerated as 143a on the bottom surface of the side view of the coupling element) which forms the bottom entrance into the interior semi-spherical volume 140a. It is understood that the head 122 of the screw 120 is held within the interior semi-spherical volume 140a by the annular rim, or support lip, 144a of the bottom 142a of the coupling element. This annular support lip 144a defines the circular opening 143a which has a diameter less than the diameter of the semi-spherical head 122 of the screw 120, but larger than the neck 124 of the screw 120 (so that the screw 120 may polyaxially rotate relative to the coupling element 132 once the head 122 has been inserted into the interior volume 140a.

In the variation of this embodiment which is shown in Figure 3a the coupling element 132 comprises a single axial slot 149 so that the element 132 has a circumferentially discontinuous conformation. This renders the coupling element 132 a 7/8ths ring, and permits the element 132 to expand to accept the inserted head portion 122, and also to crush against the head 122 upon the application of an inwardly directed radial force. Insertion of the coupling elements into the through holes prevents the coupling elements from releasing the head 122 by preventing the circular opening 143a from expanding.

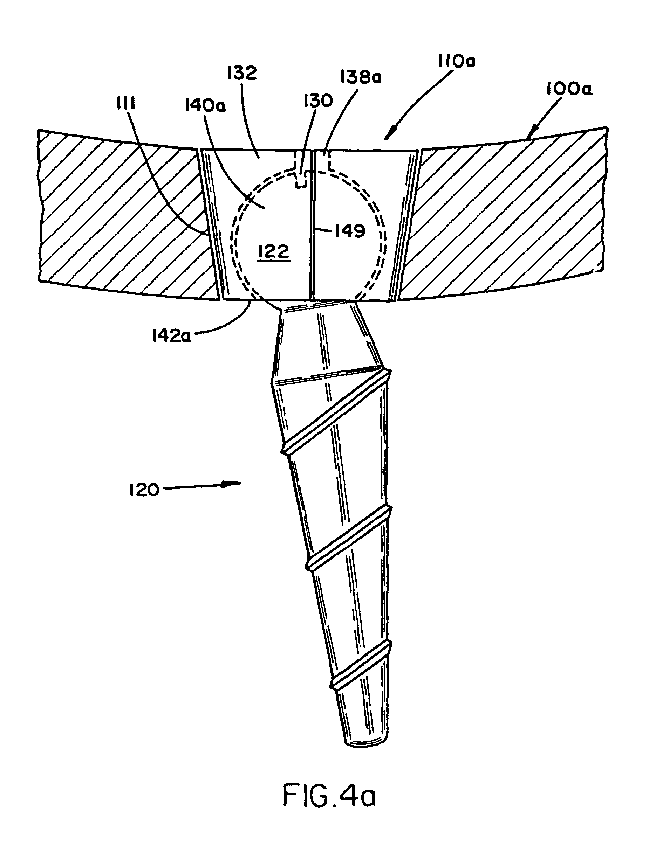

Referring now to Figure 4a, a cross sectional view of a fully assembled embodiment of the screw plate assembly of the present invention, including the smooth unitary coupling element 132 and the plate 100a having smoothly tapered holes 110a,112a, is shown. With reference to the relative positions of the screw 120, plate 100a, and coupling element 132, the operative steps of implanting this screw plate assembly and affixing it to a pair of vertebral bones begins with preparing the bones through surgical tissue exposure and/or resection. Next the plate 100a is positioned against the bones and pre-drill holes are made thereinto at the desired insertion angle for the screws 120. Each screw 120 and corresponding coupling element 132 are then joined together so that the head 122 is within the interior volume 140a, whereby the two elements are able to rotate freely with respect to one another, but are nonetheless coupled.

The recess 130 in the screw 120 and the through hole 138a of the coupling element 132 are aligned at first, and an appropriate screwdriving tool is used to insert the screw 120 through the proper hole 110a or 112a and into the pre-drilled hole in the bone. Once the screw 120 has been screwed down to the point that the bottom surface 142a of the coupling element contacts the top of the hole 110a or 112a, the coupling element 132 angulates into a proper seating position. Continued insertion of the screw 120 causes the tapered surfaces 134 and 111 of the coupling element 132 and the holes 110a, 112a, respectively, to engage. The taper 111 of the corresponding hole 110a or 112a causes the slot 149 to narrow, and the interior volume 140a of the coupling element to crush onto the surface of the head 122. Complete insertion of the coupling element 132 to the plate 100a, locks the coupling element and screw to the plate, as well as locking the screw 120 and plate 100a to the bone.

Referring now to Figures 1b, 2, 3b, and 4b, an alternative variation of the coupling element of this embodiment is shown in a side view, wherein the interior surfaces 113 of the holes 110b and 112b of the plate, as well as the exterior surfaces 137 of the corresponding coupling elements 133 are threaded.

More specifically with respect to Figure 3b, the threaded coupling element 133 is shown in a side view. This threading 139 is designed to mate with threading 113 of the holes 110b,112b of the plate 100b, so that the coupling element 133 may be screwed into the plate 100b. It is preferable that the uppermost thread 135 be designed to crush-lock so that the coupling element 133 is secure in the hole 110b.

In order for the opening 143b in the bottom 142b of the coupling element 133 to expand, thereby permitting the head 122 of the screw 120 to enter into the interior volume 140b, this variation of coupling element 133 includes a plurality of slots 146. These slots 146, as shown, extend upwardly from the bottom 142b to a point along the axial extent of the element 133 beyond the maximum diameter of the interior volume 140b. Threaded insertion of the coupling element 133 into the corresponding through hole 110b,112b causes the slots to narrow, thereby preventing the coupling element 133 from releasing the head 122 by preventing the circular opening 143b from expanding. The holes 110b,112b of the plate 100b are tapered inward such that complete insertion of the coupling element 133 thereinto causes the slots 146 to be compressed and, correspondingly, for the bottom entrance 143b and the corresponding annular lip 144b to crush lock the screw head 122 into position.

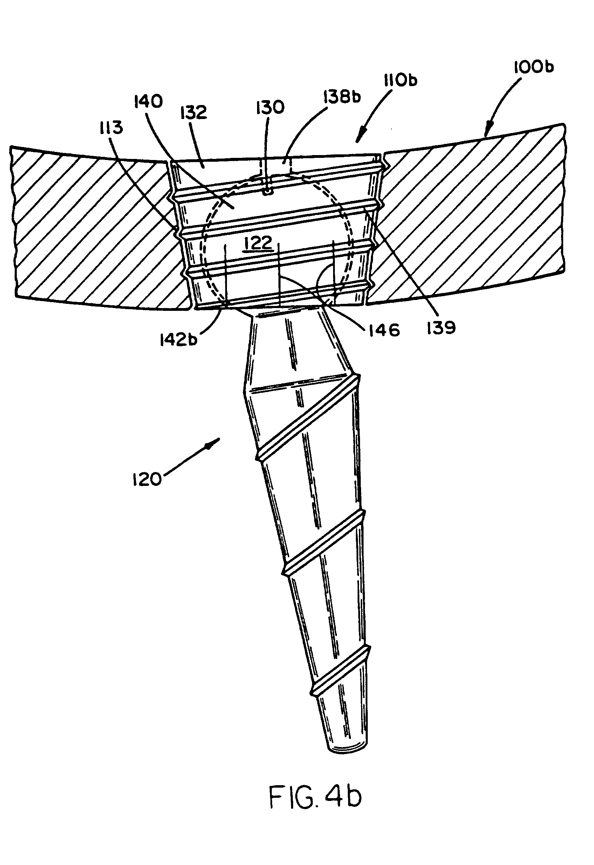

Referring now to Figure 4b, a cross sectional view of a fully assembled screw plate assembly of the present invention is shown. With reference to the relative positions of the screw 120, plate 100b, and coupling element 133, the operative steps of implanting this screw plate assembly and affixing it to a pair of vertebral bones begins with preparing the bones through surgical tissue exposure and/or resection. Next the plate 100b is positioned against the bones and pre-drill holes are made into the lateral masses at the desired insertion angle for the screws 120. Each screw 120 and corresponding coupling element 133 are then placed together so that the head 122 is within the interior volume 140b, whereby the two elements are able to rotate freely with respect to one another, but are nonetheless coupled.

The recess 130 in the screw 120 and the through hole 138b of the coupling element 133 are aligned at first, and an appropriate screwdriving tool is used to insert the screw 120 through the proper hole 110b or 112b and into the pre-drilled hole in the bone. Once the screw 120 has been screwed down to the point that the bottom surface of the coupling element 133 contacts the plate 100b, the first threads 139 of the coupling element are mated to the threading 113 of the hole 110b or 112b. Complete insertion of the coupling element 133 to the plate 100b, locks the coupling element and screw to the plate. as well as locking the screw 120 and plate 100b to the bone.

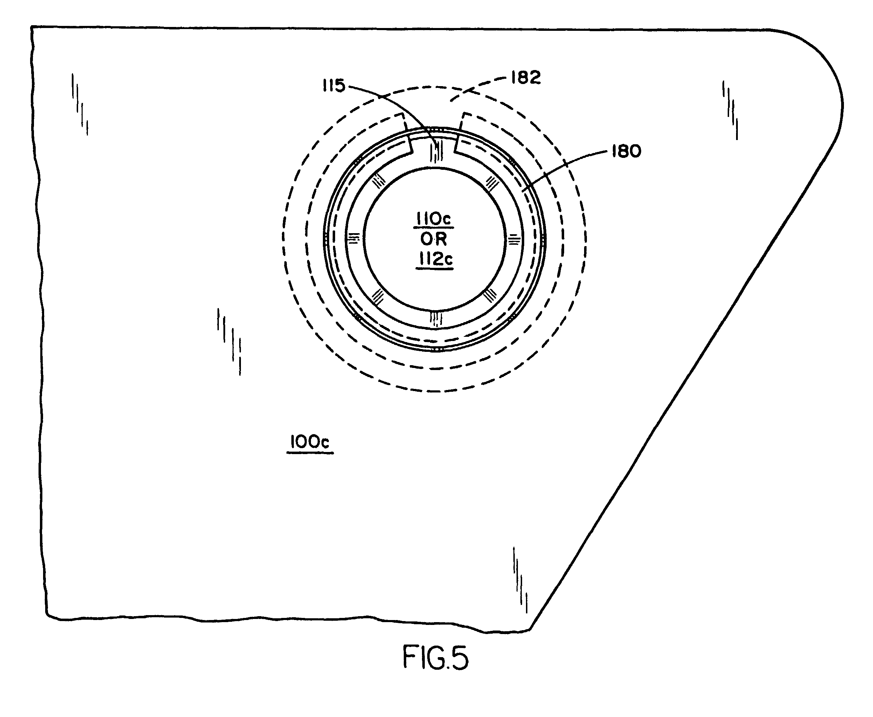

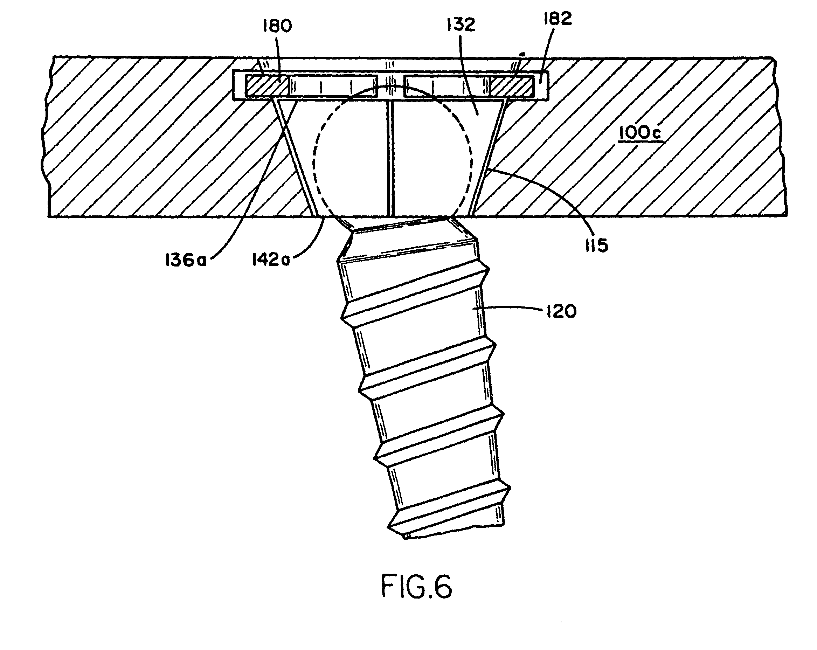

Referring now to Figures 5 and 6, a variation of the embodiments set forth above, in which there is a retaining ring provided to further lock the screw and coupling element within the tapered hole. More specifically with respect to Figure 5, which is a top view of the hole and retaining ring of this variation, the tapered hole 110c or 112c includes an annular recess formed 182 in the side wall 115 of the hole (the hole may be either smooth as in Figure 1a or threaded as in Figure 1b). Within this annular recess 182 is provided a discontinuous "7/8ths" snap-ring 180. The undeflected outer diameter of the snap-ring is greater than the diameter of the hole 110c or 112c (so that it will remain in the recess), and is also less than the diameter of the top 136a or 136b of the coupling element 132 or 133, respectively. The inner diameter of the undeflected snap-ring 180 is, however, less than the diameter of the tapered hole, but is greater than the bottom 142a or 142b of the coupling element.

Referring now also to Figure 6, the assembled screw 120, coupling element 132. snap-ring 180 and plate 100c are shown. The snap-ring 180 is deflected inward for positioning in the recess 182. During the implantation procedure, the screw 120 and the coupling element 132 (or 133) are inserted through the hole. Once the bottom 142a (or 142b) of the coupling element 132 (or 133), respectively, seats in the top of the hole 110c or 112c, and begins to travel into it, the tapered exterior surface of the coupling element 132 (or 133) causes the snap-ring 180 to expand into the recess 182. Once the coupling element 132 (or 133) is fully seated in the hole 110c, the snap-ring 180 is freed from the outward radial pressure of the coupling element 132 (or 133) and snaps back to its undeflected state. In its undeflected state it prevents the coupling element 132 (or 133) from backing out of the plate 100c inasmuch as the flat upper surface 136a (or 136b) of the coupling element is incapable of deflecting the ring outward (it has no taper to push the snap-ring open).

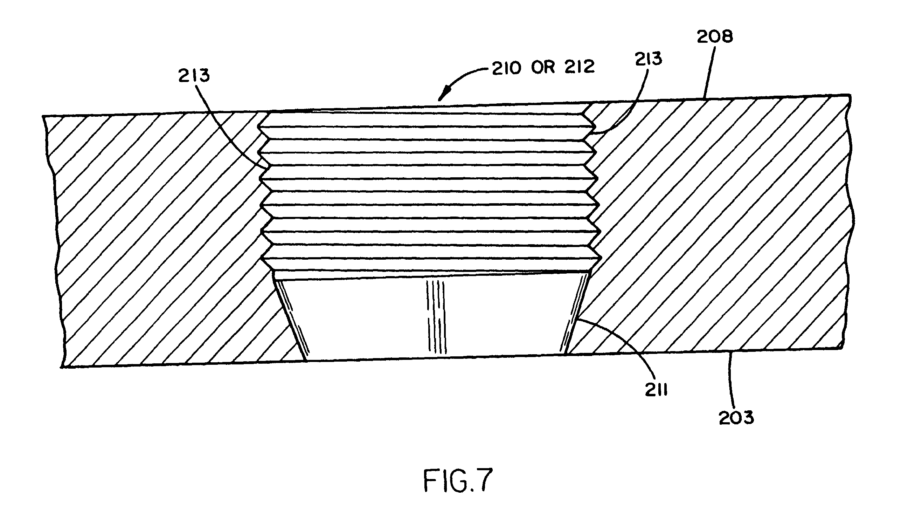

Referring now to Figure 7, a cross-section view of the hole 210 or 212 in plate 200, which otherwise similar to the plates 100a and 100b shown in Figures 1a and 1b, is shown. Extending from the top surface 208 of the plate, into the hole 210 or 212, to a position which is above the bottom surface 203 is a threading 213. The diameter of the hole 210 or 212 does not vary in this threaded region 213, however, the bottom section of the hole includes an inward taper 211 and is smooth.

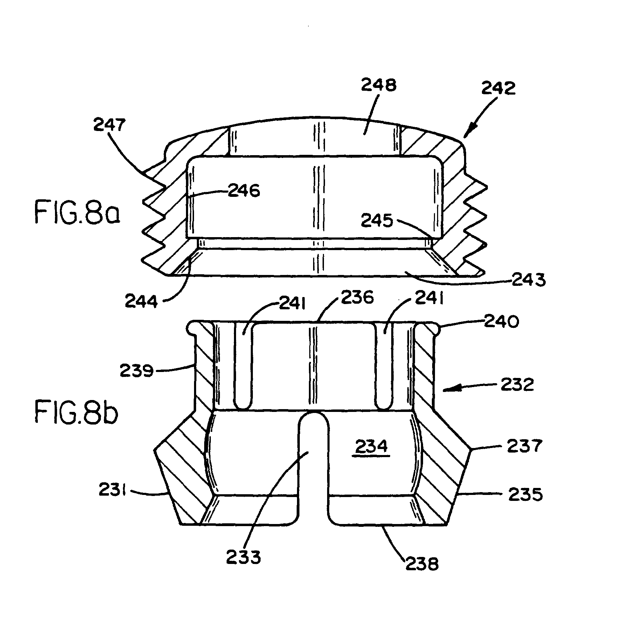

Referring now to Figures 8a and 8b, the two portions which form the coupling element of the present invention are shown in side cross-section views. Phantom lines show the interior structure of the elements along the diametrical cross section. With specific reference to the socket portion 232, shown in Figure 8b. the coupling element comprises a roughly cylindrical shape having an interior volume 234 in which the semi-spherical head 122 of the screw 120 is disposed. The interior volume 234 is open at the top 236 of the socket portion 232 and at the bottom thereof 238. The lower section 231 of the socket portion 232 comprises a set of slots 233 which extend vertically from the bottom 238 of the socket portion 232 to a position above the maximum diameter of the semi-spherical interior volume 234. These slots 233 permit the interior volume to expand and contract in accordance with the application of a radial force thereon. The external surface 235 of the lower section 231 of the socket portion 232 is tapered such that the narrowest part of the lower section 231 is at the bottom 238.

The upper section 239 of the socket portion 232, shown in Figure 8b, has a generally constant diameter, which is less than the diameter at the uppermost position 237 of the taper of the lower section 231. A second set of vertical slots 241 are provided in this upper section 239 so that it may also expand and contract in accordance with radial forces applied thereto. In addition, the uppermost end of this upper section 239 comprises an outwardly extending annular lip 240.

The cap portion 242 of the coupling element, shown in Figure 8a, comprises an opening 243 in the bottom thereof, having an inwardly tapered entrance surface conformation 244. As the upper section 239 of the socket portion 232 is inserted into the opening 243 in the cap portion 242, the taper 244 of the opening 243 provides an inwardly directed force which causes the upper section 239 to contract (causes the slots 241 to narrow). This tapered entrance 244 opens to form an annular lip 245 which is useful for engaging and retaining the annular lip 240 of the upper section 239 of the socket portion 232. The interior surface 246 of the cap portion has a constant diameter, therein permitting the inserted upper section 239 of the socket portion 232 to slide and rotate relative to the cap portion 242.

The exterior surface of the cap portion 242 comprises a threading 247 which is designed to engage the threading 213 in the upper portion of the corresponding hole 210 or 212. In addition, the cap portion 242 comprises an axial hole 248 through which a surgeon may insert a screw driving tool to access the head of the screw which is positioned in the interior volume 234 of the socket portion 232.

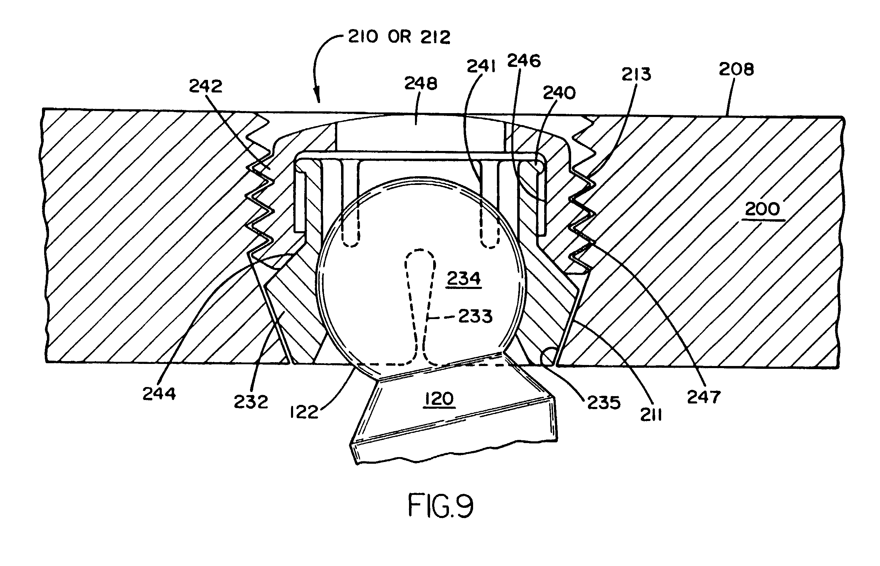

More particularly, with respect to the disposition of the head 122 of the screw 120 in the socket portion 232, and with reference to Figure 9, a fully assembled plate, coupling element, and screw is shown in a side cross-section view. The top 236 of the socket portion 232 is inserted into the opening in the cap portion 242 until the annular lip 240 of the socket 232 seats into the cap 242. The screw 120 is loosely held within the socket 232, which is, in turn, loosely retained within the cap 242.

With respect to the imploantation of the device, as with the first two embodiments, the plate 200 is positioned against the vertebral bones which are to be immobilized. A drill is used to pre-form holes into which the bone screws 120 are to be inserted (at the desired angulation). The screw 120 is then inserted and driven downward into the bone by use of the appropriate screw driving tool. As the screw 120 is driven deeper into the bone, the coupling element mounted to the head of the screw begins to enter the hole 210 or 212 of the plate 200. As the coupling element enters the hole 210 or 212, the tapered exterior surface 235 of the socket portion 232 seats against the tapered bottom 211 of the hole 210 or 212. Continued driving of the screw into the bone causes the slot 233 in the bottom of the socket portion 232 to narrow, thus causing the interior volume 234 of the socket portion 232 to crush against the head 122 of the screw 120.

The cap portion 242 of the coupling element may then be threadably advanced into the top section of the hole. As it is advanced, the upper annular lip 240 of the socket portion 232 slides upwardly along the inner surface 246 of the cap 242 until the bottom tapered opening 244 contacts the widened taper position of the socket portion 232. Continued advancement of the cap portion 242 provides further advancement of the socket portion 232 into the hole 210 or 212, thereby increased locking pressure within the interior volume 234 against the head 222.

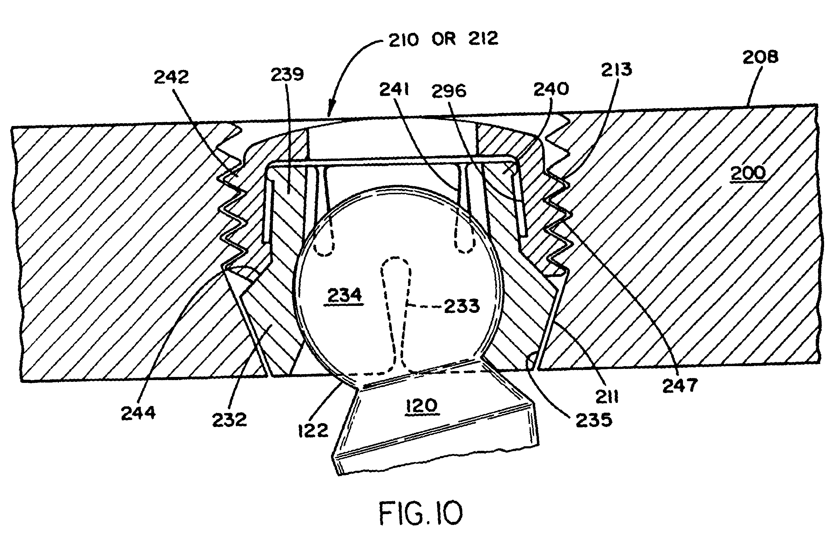

Referring to Figure 10, a variation of this two-piece coupling element device is shown in a similar cross-section view. In this embodiment, the inner surface 296 of the cap portion 242 is tapered inwardly in the vertical direction so that the advancement of the cap portion 242 along the threading 213 of the hole 210 or 212 causes the annular lip 240 to be compressed inwardly. This causes the slots 241 of the upper section 239 of the socket portion 232 to narrow. This may be utilized to further clamp the interior volume 234 against the head 122 of the screw 120. Once screwed into the plate 200, and locked down, the cap portion 242 of the coupling element and the top surface of the plate 208 present a flush external surface.

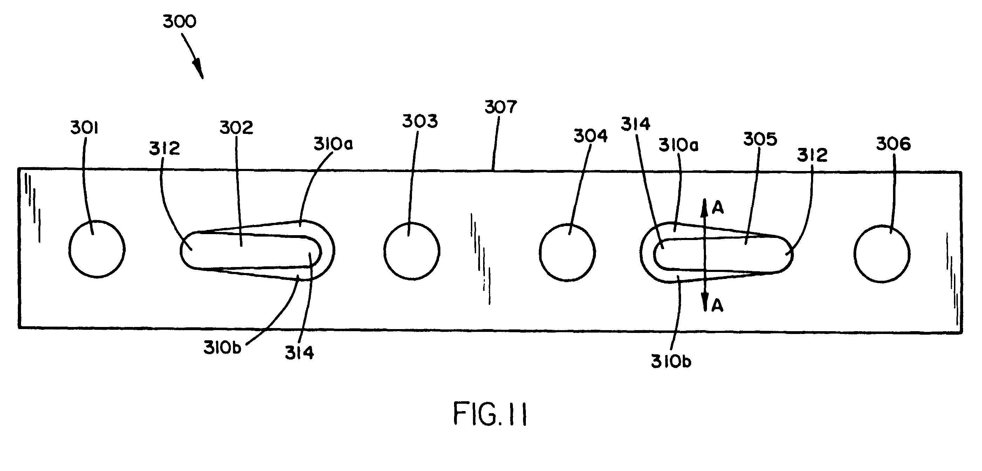

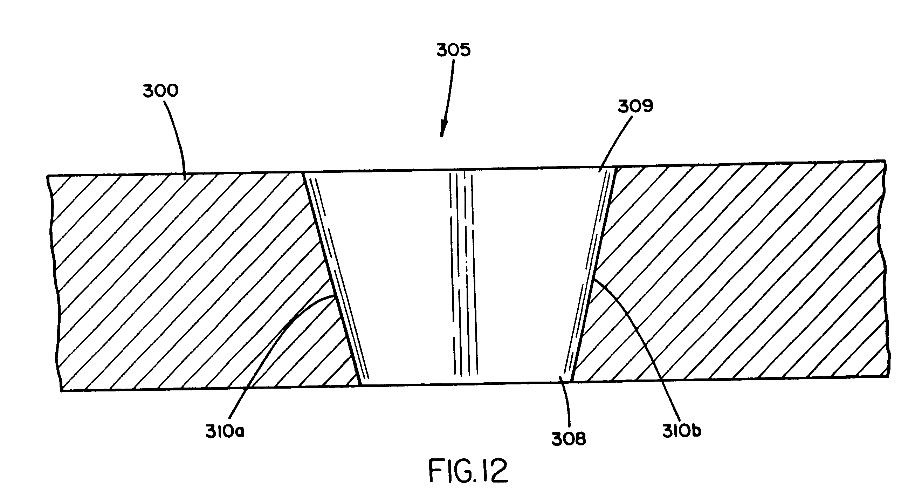

Referring now to Figure 11, a top view of the plate of the first embodiment of the present invention is shown wherein axis A-A is provided as a reference to a subsequent cross-section view provided in Figure 12. The plate 300 comprises an longitudinally extended, generally rectangular, thin metal plate, having a plurality of holes 301-306 defined therein. The holes 301-306 are divided into two groups of three, sequentially and symmetrically disposed on either sides of the longitudinal middle 307 of the plate. Holes 301, 303, 304, and 306 are provided for holding screws which provide additional securing strength once the initial affixation of the assembly, and the compression associated therewith, has been carried out. These holes and the corresponding screws are preferably of one of the types described above.

Holes 302 and 305 are disposed on either side of the middle 307 of the plate 300, and are preferably equidistant therefrom. The holes 302 and 305 are oriented in opposite sense with respect to the middle 307 of the plate 300, such that remote ends 312 thereof are farther from the middle 307 than the proximal ends 314. These holes 302 and 305 are elongated in the longitudinal direction of the plate 300. having tapered sidewalls 310a,310b.

More specifically with respect to the sidewall morphology, and referring also to Figure 12, the sidewalls 310a and 310b of the elongate hole 305 (or 302) are tapered along axes which are perpendicular to both the plane of the plate 300, and to the elongated sense of the hole 305. The width of the hole 305 is narrower at the bottom 308 than at the top 309. The taper of the hole 305 is such that the separation of the side walls 310a,310b is wider at the proximal end 314 than at the remote end 312.

The coupling element used in conjunction with this type of elongate hole is equivalent to the smooth tapered unitary coupling element 132 shown in Figure 3a. The exterior surface of the element 132 mates with the surfaces morphology of the elongate holes 302,305. More specifically, the narrower bottom 142a of the coupling element 132 is slightly smaller than the distance between the sidewalls 310a,310b of the top 309 of the elongate hole, at the remote end 312, which is in turn considerably narrower than the top 136a of the coupling element. The tapered sidewalls 310a,310b of the plate 300 widen in the direction of the middle 307, such that the distance between the sidewalls 310a,310b of the elongate hole 305 at the proximal end 314, are only slightly smaller than the corresponding tapered axial diameter of the coupling element 132. At the proximal end 314, it is this slight difference in tapering which provides the compression lock of the coupling element 132 to both the screw head 120 and the plate 300.

The first step in the process of implanting this embodiment of the invention is to position the plate against the segments of the broken bone which require compression and immobilization, and to align the entry points for the screws. The next step is to pre-drill holes into the bones at the desired position along the elongate hole, and angle with respect to the plate, into which the screws will be inserted. With the plate in place, one screw 120 and corresponding coupling element 132 are inserted through respective elongate holes 302,307 into the bone segments. In this embodiment, the screws may be inserted at any point along the length of their corresponding holes 302,305.

Once the screw has been inserted into the bone, at the desired angle, the coupling element 132, via its rotationally free mating of the element 132 to the head 122 of the screw, is realigned so that it may be seated against the tapered surfaces 310a,310b, in the elongated hole 302,305. If the screw is inserted at a point which is not directly in the proximal end 314, for example at the extreme other end, the remote end 314, the seating of the coupling element 132 is not as deep as it could be. Continued rotation of the screw 120, therefore, causes the coupling element 132 to slide deeper into the hole, which correspondingly requires that the screw 120 and bone move relative to the plate 300. The coupling element 132 continues to slide down the ever widening tapered walls 310a,310b of the hole, until it reaches the proximal end 314. At this proximal end 314, as the sidewalls 310a,310b remain slightly narrower than the coupling element 132, continued driving of the screw into the bone causes the slot 149 to close, therein crush locking the coupling element 132 to the screw head 122, and further therein compression locking the coupling element to the plate.

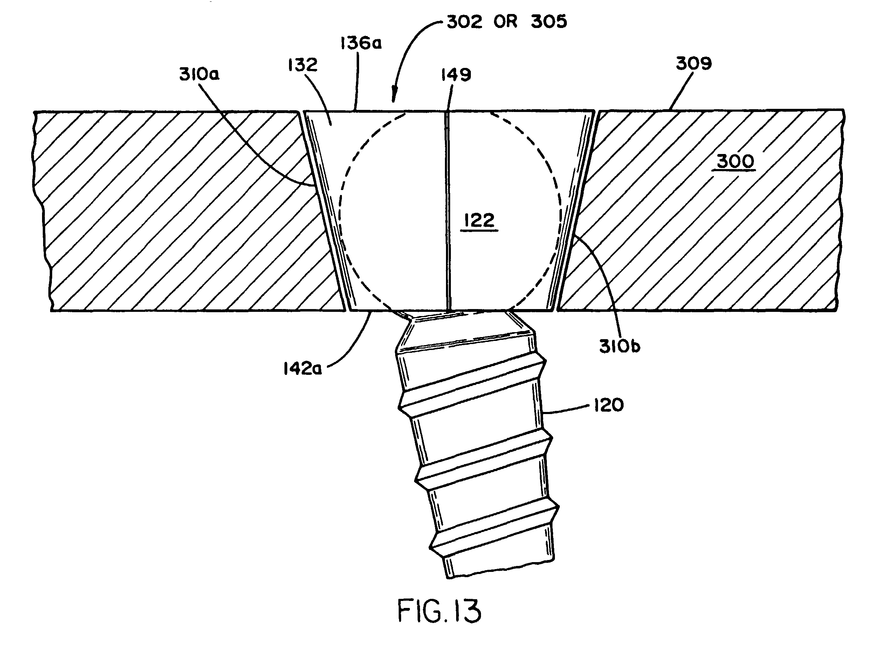

Referring now to Figure 13, a cross-section view of the fully seated and locked screw 120 and coupling element 132 is shown. As stated above, and shown herein, the side walls 310a,310b of the elongate hole 302,305 are slightly narrowed so that the slot 149 is narrowed, therein crush locking the head 122 to the element 132, and compression locked to the plate 300.

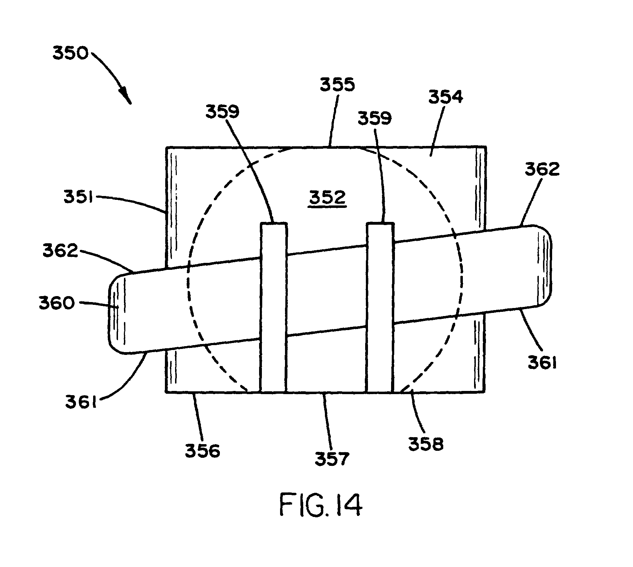

Referring now to Figure 14, the coupling element 350 of a further embodiment of the dynamic compression plate variation of the present invention is shown in a side view. As in the coupling element 132 of the first embodiment, the body 351 of the element comprises an interior volume 352, into which the semi-spherical head 122 of a screw 120, such as is shown in Figure 2, may be polyaxially disposed. The top 354 of the coupling element comprises a hole 355 through which the surgeon may access the screwdriving hole in the head 122 of the screw 120. The bottom 356 of the coupling element 350 comprises a hole 357 through which the head of the screw may be inserted. The hole 357 has a diameter which is less than the major diameter of the screw head 122, so that the screw may be held therein. The mouth of the bottom hole 357, therefore, includes an annular lip 358 which prevents the ball head from escaping the inner volume 352. In order to provide a means by which the screw head 122 may be inserted into the coupling element 350, the bottom of the element 350 may include slots 359 which expand under the application of a radial force outward, therein expanding the mouth of the hole 357 to receive a larger diameter object therethrough. The slots 359 further permit the bottom 356 of the coupling element 350 to be compressed inward, locking the element to the head 122 of the screw, upon the application of a radially inward force. (It shall be understood that the slots may alternatively descend from the top 354 of the element 350. In such an embodiment, the screw 120 would be inserted axially through the coupling element 350 from the top, and the top opening 355 would expand to receive the ball head 122 of the screw. This embodiment would lock the screw 120 in interior volume 352 of the coupling element by narrowing the slots in the same manner as the embodiment shown in Figure 14.)

The coupling element further comprises an annular flange portion 360 which extends outward from the cylindrical body. The flange 360 defines a circular ring which is offset slightly with respect to the axis of the coupling element body 351. The flange 360 has parallel upper and lower surfaces 362 and 361, respectively, and is ideally suited for travelling within a pair of parallel sloped channels as are described hereinbelow with respect to the corresponding plate of this embodiment.

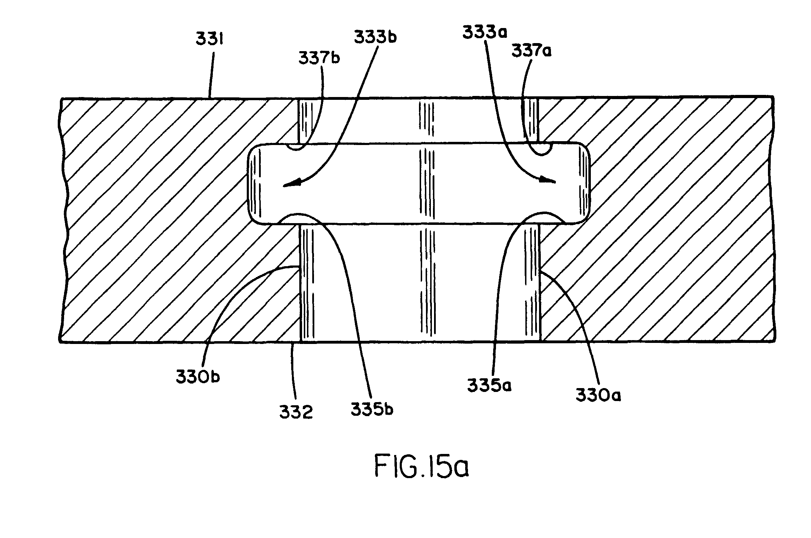

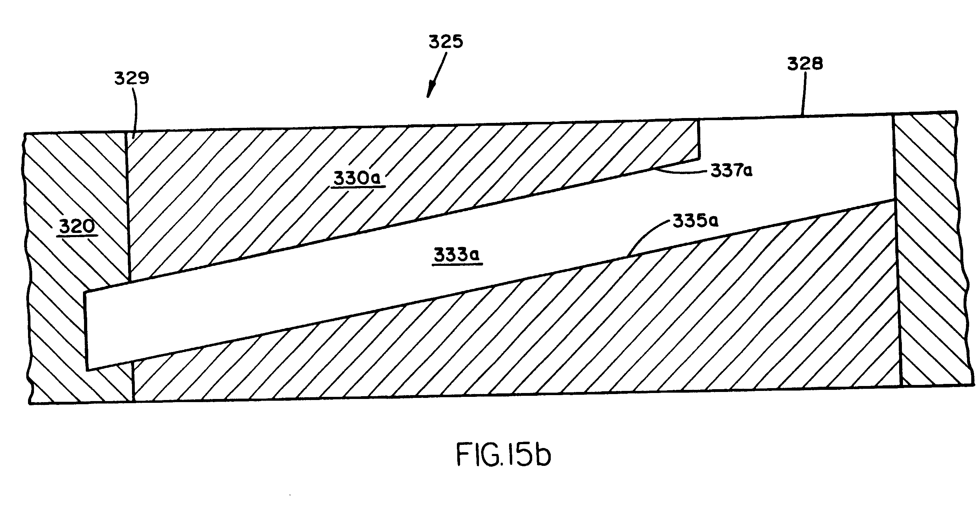

Referring now to Figures 15a and 15b, the plate 320 (and an elongate dynamic compression hole) of this embodiment of the present invention is shown in two mutually perpendicular cross-sectional views. More particularly, Figure 15a shows the a cross-section view of the hole which is transverse to the axis of the hole, and illustrates that the parallel sidewalls 330a,330b of the hole comprise, respective, recessed channels 333a,333b. The channels 333a,333b include defining lower ledges 335a,335b and upper ledge 337a,337b. Figure 15b shows an axial cross-section view of the hole, and illustrates that the channels 333a,333b are sloped downward along the sidewalls 330a,330b. At remote end 328 the channels 333a,333b are open so that the lower ledge 335a,335b may receive thereon the lower side 361 of the offset annular flange 360 of coupling element 350. The offset angulation of the flange 360 is ideally set to match the angulation of the sloped channels 333.

The initial steps of the implantation of the second embodiment of the present invention, including the preparation of the bone and the pre-drill holes, are similar to the preparatory steps associated with the first embodiment. Once positioned properly, the screws 120 are inserted in the bone through their respective elongate hole until the corresponding coupling elements 350 have properly seated in the remote ends 328 of the elongate holes, with the lower surface 361 of the flanges 360 of each element 350 disposed on the lower ledges 335a,335b of the channels 333a,333b. From this point further, continued driving of the screw into the bone causes the flange 360 of the coupling element 350 to slide down the sloped channels 333a,333b until the head has reached the deepest point at the proximal end 329. This translation of the coupling element down the channels 333a,333b. necessarily causes the bone into which the screw has been inserted to move relative to the fixed plate. As the process is carried out in opposing directions by the oppositely oriented elongate holes, displaced bone segments may be brought together and held fixed in contact.

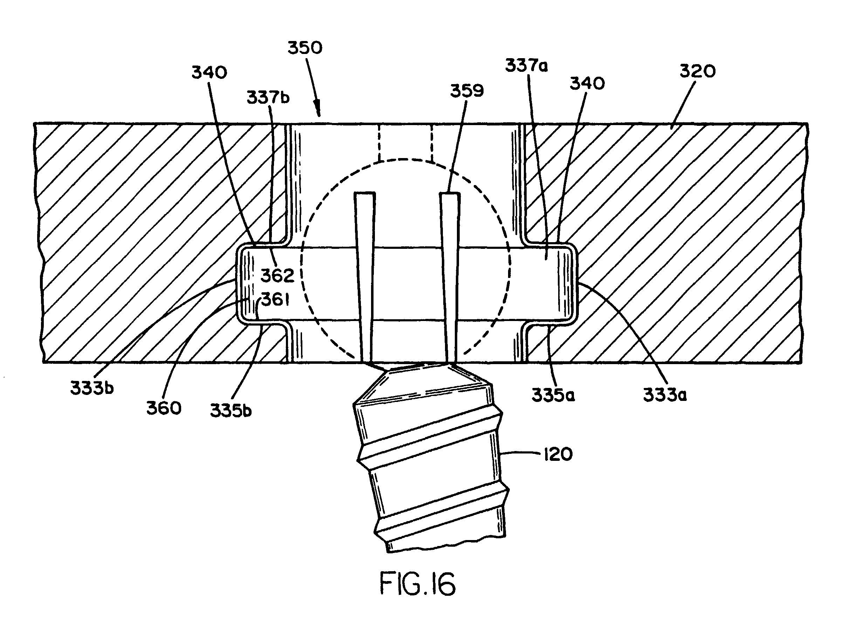

In a preferred variation, the channels 333a,333b narrow slightly along the longitudinal direction of the elongate hole, in the direction perpendicular to the axial direction of the elongate hole. This slight narrowing provides an inwardly directed radial force which causes slots 359 to close, thus locking the polyaxial screw 120 to the coupling element 350, so that it does not rotate with respect thereto. In all variations, however, it is preferred that the upper ledges 337a,337b of each channel 333a,333b in each of the sidewalls 330a,330b provide contact surfaces against which the upper surface 352 of the offset annular flange 360 interfaces. This interface 340, as shown in Figure 16, which illustrates the screw 120 and coupling element 350 in their fully inserted position, prevents screw back-out. In some variations, however, the upper ledges 337a,337b are only provided at the proximal ends of the elongate holes so that the screws 120 and coupling elements 350 may be inserted at any point along the extent of the holes.

More specifically, Figure 16 is provided to illustrate the coupling element 350 fully inserted in the plate 320, with offset annular flange 360 (which appears parallel in this cross-section angle) fully nested between the upper and lower ledges 362,361, respectively, of the channels 333a,333b.

While there has been described and illustrated implantation devices for stabilizing and immobilizing bone segments by affixing a polyaxial locking screw plate thereto, it will be apparent to those skilled in the art that variations and modifications are possible without deviating from the broad principle of the present invention which shall be limited solely by the scope of the claims appended hereto.

## Claims

1. An orthopaedic implantation device which locks a polyaxial screw (120) to an implant structure through which it is inserted comprising:
a plate (100a) having a plurality of through holes (110a, 112a),
a plurality of bone screws (120) each having a head portion (122) the head portion being semi-spherical and a shaft (126), the shaft (126) portion being insertable through a corresponding through hole (110, 112) in the plate (100a) and into the implant structure **characterised in that** the device also comprises:
a plurality of coupling elements (132), each being insertable into a corresponding one of the though holes (110a, 112a), the coupling element (132) having an interior receiving volume (140a), shaped to retain the semi-spherical head portion and being adapted such that the interior volume (140a) may expand or contract by the application of a radial force to receive and/or lock the head portion (122) of the screw (120) at the desired angle in the implant structure, whereby the head portion (122) is rotationally freely mounted in the coupling element (132) prior to insertion, and the shaft (126) of the bone screw (120) and the coupling element (132) may be inserted into a corresponding through hole (110a, 112a) and the shaft (126) of the bone screw may be inserted into the bone at a selected angle within a predetermined range of angles including non-perpendicular angles relative to the plate.

2. A device as claimed in claim 1, **characterised in that** the coupling element (132) further comprises at least one axial slot (149) which permits the interior volume (140a) to expand thereby facilitating the insertion of the head portion (122) of the screw therein, and to contract on insertion of the coupling element (132) into the corresponding through hole (110a, 112a) thereby locking the head portion (122) within the interior volume (140a) of the coupling element.

3. A device as claimed in claim 2, **characterised in that** the axial slot (149) extends the entire length of the coupling element (132).

4. A device as claimed in any preceding claim **characterised in that** the coupling elements (132) each comprise an interior semi-spherical receiving volume (140a), defined by a curved interior surface, which forms a receiving socket into which the semi-spherical head portion (122) is insertable.

5. A device as claimed in any preceding claim, **characterised in that** the coupling element (132) has an axially tapered exterior surface (134) and the corresponding through hole (110a, 112a) into which the coupling element (132) is insertable has a corresponding inward taper, thereby causing, upon insertion of the coupling element (132) into the corresponding though hole (110a, 112a), the at least one slot (149) to be narrowed, causing the curved interior surface of the coupling element (132) to lock the semi-spherical head portion (122) of the screw (120) at a definite insertion angle.

6. A device as claimed in any preceding claim, **characterised in that** the head portion (122) of each of the screws (120) comprises a recess (130) into which a screwdriving tool is matable for inserting the screw (120) though the corresponding hole (110, 112) and into the bone.

7. A device as claimed in any preceding claim, **characterised in that** each of the coupling elements (132) comprises a top surface recess (138a), though which the screwdriving tool may be inserted, and the top surface recess (138a) being aligned with the recess (130) in the head of the screw (120), such that a screwdriver may insert the screw (120) into the bone.

8. A device as claimed in any preceding claim **characterised in that** at least one of the plurality of through holes (110a, 112a) further comprises an annular recess (182) formed in an upper sidewall (115) portion of the hole, and a snap-ring (180) located in the recess (182), the snap-ring (180) deflecting inward during the insertion of the coupling element (132) to permit the coupling element to travel into the though hole (110a, 112a), and the snap-ring (180) snapping back to an undeflected condition once the coupling element (132) has fully seated in the though hole (110a, 112a), thereby preventing the coupling element (132) from backing out of the through hole (110a, 112a).

9. A device as claimed in any preceding claim **characterised in that** the plate (100a) includes a first end and a second end, a first group (110a) of the though holes being disposed at the first end, and a second group (112a) of the though holes being disposed in the second end.

10. A device as claimed in any preceding claim **characterised in that** the through holes (110b, 112b) include an interior surface threading, and each of the coupling elements (132) includes an exterior surface threading which is matable to the interior surface threading of the through holes (110b, 112b), whereby each of the coupling elements (132) is insertable into the corresponding through hole (110b, 112b) via engagement of the interior and exterior surface threadings.

11. A device as claimed in any preceding claim **characterised in that** :
the coupling elements (132), each comprise
a socket portion (232) having an interior volume (234), upper (239) and lower (231) sections, and at least one vertical slot (233, 241) formed on corresponding ones of the upper and lower sections, at least one of the slots (233) rendering the interior volume (234) expandable and contractible, the lower section (231) having a tapered exterior surface (235) for seating against the lower tapered portion of the corresponding though hole (210, 212,) such that forcible advancement of the socket portion (232) into the tapered portion of the through hole (210, 212) causes the at least one of the slots (233) to narrow whereby the interior volume contracts, and
a cap portion (242) having an opening (243) in a bottom thereof and an interior chamber extending upwardly therefrom for joining with, and slidably retaining therein, the upper section of the socket portion (233), and exterior threading (247) which is mateable with the threading of the corresponding hole,
such that the head portion (122) of the bone screw is rotationally freely mounted within the interior volume of the socket portion (232) of the coupling element (132) prior to insertion, and the insertion of the shaft (126) of the bone screw and the coupling element into the corresponding through hole (210, 212) and the insertion of the shaft (126) of the bone screw at a selected angle causes the tapered exterior surface of the socket portion (233) to seat and to be forcibly advanced against the tapered portion of the through hole (210, 212) such that the head (122) of the screw is locked within the contracted semi-spherical interior volume, and threaded advancement of the cap portion (242) into the though hole (210, 212) provides additional forcible advancement of the socket portion (232) against the tapered portion of the though hole.

12. A device as claimed in claim 11, **characterised in that** the socket portion (232) comprises a substantially constant diameter upper section having an outwardly annular extending lip (240) at an extreme end thereof,
and **in that** the opening (243) in the bottom of the cap portion (242) comprises an inwardly directed annular lip (245), and
at least one of the vertical slots (233) in the upper section of the socket portion (232) renders the upper section thereof to be expandable and contractible such that the upper section of the socket portion (232) may be forcibly inserted into the opening (243) in the bottom of the cap portion (242) so that it may be retained in the interior chamber therein by manual interference engagement of the inwardly directed annular lip (245) of the cap portion and the outwardly extending annular lip (240) of the socket portion.

13. A device as claimed in claim 11 or 12, **characterised in that** the interior chamber of the cap portion comprises a tapered surface (244) such that advancement thereof into the hole (210, 212) causes an inwardly directed force against the upper section of the socket portion (232), therein causing the at least one of the vertical slots (241) in the upper section to narrow and causes the upper section to contract and further lock the head (122) of the screw within the interior semi-spherical volume of the socket portion (234).

14. A device as claimed in any preceding claim **characterised in that**
said plate is an elongate plate (300) including first and second longitudinal portions, and a middle (307) defined therebetween; said device further comprising
at least one elongate hole (302, 305), extending though at least one of the first and second longitudinal portions, having a remote end (312) and a proximal end (314) thereof and lateral sidewalls (310a, 310b) connecting the ends, each of the sidewalls including a sloped surface conformation;
means in at least the other of the first and second longitudinal portions by which the plate is securable to a first one of the plurality of the bone segments;
at least one corresponding coupling element (132) being insertable into the at least one elongate hole (302, 305), slidable along the sloped surface conformations, and lockable within the elongate hole.

15. A device as claimed in claim 14 **characterised in that** each of the longitudinal portions of the plate comprises at least one elongate hole (302, 305).

16. A device as claimed in claim 14 or 15, **characterised in that** the plate further comprises at least one round hole (301, 303, 304, 306), and the means comprises a holding screw (120), insertable though the at least one round hole to provide added fixation strength.

17. A device as claimed in any of claims 14 to 16, **characterised in that** the at least one elongate hole (302, 305) extends in the direction of the elongate axis of the plate, the sidewalls (310a, 310b) being tapered in a first axis perpendicular to the plane of the plate (300), and in a second axis coaxial with the hole (302, 305) itself, such that the hole (302, 305) is narrower at the bottom than the top and the separation of the side walls (310a, 310b) is wider at the proximal end (314) than the remote end (312),
and the coupling element (132) is cylindrical and tapered to form a frusto-conical section,
the external conformation permitting lockable mating with the sidewall morphology of the corresponding elongate hole (302, 305).

18. A device as claimed in any of claims 14 to 16, **characterised in that** the at least one elongate hole (302, 305) extends in the direction of the elongate axis of the plate (300), the sidewall (310a, 310b) of the hole comprising at least one sloped channel (333a, 333b), the channel comprising a recessed lower lip (335a, 335b)which forms the lower extent of the channel which is sloped downward into the plate and an upper lip portion (337a, 337b) which defines the upper extent of the channel, the remote end (328) of the channel being open,
and a coupling element (350) which comprises an annular flange (360) extending outwardly from the body of the element and seatable on the lower lip (335a, 335b) of the elongate hole and driveable by continued insertion of the screw (120) down the slope of the channel.

## Patentansprüche

1. Orthopädische Implantationsvorrichtung, die eine polyaxiale Schraube (120) mit einer Implantatstruktur verbindet, durch welche sie geführt ist, mit:
- einer Platte (100a) mit mehreren Durchgangslöchern (110a, 112a),
- mehreren Knochenschrauben (120) mit einem halbkugelförmigen Kopfbereich (122) und einem Schaft (126), wobei der Schaftbereich (126) durch ein entsprechendes Durchgangsloch (10, 112) in der Platte (100a) und in die Implantatstruktur einführbar ist, **dadurch gekennzeichnet, daß** die Vorrichtung ferner aufweist:
- mehrere Verbindungselemente (132), die jeweils in ein entsprechendes Durchgangsloch (110a, 112a) einführbar sind, wobei das Verbindungselement (132) ein zum Halten des halbkugelförmigen Kopfbereichs ausgebildetes inneres Aufnahmevolumen (140a) aufweist und derart ausgebildet ist, daß das innere Volumen (140a) sich durch Aufbringen einer radialen Kraft erweitert oder zusammenzieht, um den Kopfbereich (122) der Schraube (120) unter dem gewünschten Winkel im Implantat aufzunehmen und/oder zu blockieren, wodurch der Kopfbereich (122) vor dem Einführen frei drehbar im Verbindungselement (132) angebracht ist, und der Schaft (126) der Knochenschraube (120) und das Verbindungselement (132) in ein entsprechendes Durchgangsloch (110a, 112a) eingesetzt werden können und der Schaft (126) der Knochenschraube in den Knochen unter einem gewählten Winkel innerhalb eines vorbestimmten Winkelbereichs, der zur Platte nicht senkrechte Winkel umfaßt, eingesetzt werden kann.

2. Vorrichtung nach Anspruch 1, **dadurch gekennzeichnet, daß** das Verbindungselement (132) ferner wenigstens einen axialen Schlitz (149) aufweist, der ein Aufweiten des Innenvolumens (140a) ermöglicht, wodurch das Einführen des Kopfbereichs (122) der Schraube in dieses erleichtert ist, und ein Zusammenziehen beim Einsetzen des Verbindungselements (132) in das entsprechende Durchgangsloch (110a, 112a) ermöglicht, wodurch der Kopfbereich (122) im Innenvolumen (140a) des Verbindungselements blockiert ist.

3. Vorrichtung nach Anspruch 2, **dadurch gekennzeichnet, daß** der axiale Schlitz (149) sich über die gesamte Länge des Verbindungselements (132) erstreckt.

4. Vorrichtung nach einem der vorhergehenden Ansprüche, **dadurch gekennzeichnet, daß** die Verbindungselemente (132) jeweils ein durch eine gebogene Innenfläche begrenztes inneres halbkugelförmiges Aufnahmevolumen (140a) aufweisen, welches eine Aufnahmebuchse bildet, in die der halbkugelförmige Kopfbereich (122) einsetzbar ist.

5. Vorrichtung nach einem der vorhergehenden Ansprüche, **dadurch gekennzeichnet, daß** das Verbindungselement (132) eine sich axial verjüngende Außenfläche (134) und das entsprechende Durchgangsloch (110a, 112a), in welches das Verbindungselement (132) einsetzbar ist, eine entsprechende einwärts gerichtete Verjüngung aufweist, wodurch beim Einsetzen des Verbindungselements (132) in das entsprechende Durchgangsloch (110a, 112a) eine Verengung des wenigstens einen Schlitzes (149) bewirkt wird, wobei die gebogene Innenfläche des Verbindungselements (132) das Blockieren des halbkugelförmigen Kopfbereichs (133) der Schraube (120) in einem endgültigen Einführwinkel bewirkt.

6. Vorrichtung nach einem der vorhergehenden Ansprüche, **dadurch gekennzeichnet, daß** der Kopfbereich (122) jeder der Schrauben (120) eine Ausnehmung (130) aufweist, in die ein Schraubendreherwerkzeug einsetzbar ist, um die Schraube (120) durch das entsprechende Loch (110, 112) und in den Knochen einzuführen.

7. Vorrichtung nach einem der vorhergehenden Ansprüche, **dadurch gekennzeichnet, daß** jedes der Verbindungselemente (132) eine Oberseitenausnehmung (138a) aufweist, durch welche das Schraubendreherwerkzeug eingeführt werden kann, und wobei die Oberseitenausnehmung (138a) mit der Ausnehmung (130) im Kopf der Schraube (120) fluchtet, so daß die Schraube (120) mittels eines Schraubendrehers in den Knochen einsetzbar ist.

8. Vorrichtung nach einem der vorhergehenden Ansprüche, **dadurch gekennzeichnet, daß** wenigstens eines der mehreren Durchgangslöcher (110a, 112a) ferner eine in einem oberen Seitenwandbereich (115) des Lochs ausgebildete ringförmige Ausnehmung (182) und einen in der Ausnehmung (182) angeordneten Schnappring (180) aufweist, der beim Einführen des Verbindungselements (132) nach innen ausgelenkt wird, um den Durchtritt des Verbindungselements in das Durchgangsloch (110a, 112a) zu ermöglichen, wobei der Schnappring (180) in den nicht ausgelenkten Zustand zurückschnappt, sobald das Verbindungselement (132) vollständig im Durchgangsloch (110a, 112a) sitzt, wodurch das Zurückbewegen des Verbindungselements (132) aus dem Durchgangsloch (110a, 112a) verhindert ist.

9. Vorrichtung nach einem der vorhergehenden Ansprüche, **dadurch gekennzeichnet, daß** die Platte (100a) ein erstes Ende und ein zweites Ende aufweist, wobei eine erste Gruppe (110a) von Durchgangslöchern am ersten Ende und eine zweite Gruppe (112a) von Durchgangslöchern am zweiten Ende angeordnet ist.

10. Vorrichtung nach einem der vorhergehenden Ansprüche, **dadurch gekennzeichnet, daß** die Durchgangslöcher (110b, 112b) ein Innengewinde und jedes der Verbindungselemente (132) ein Außengewinde aufweist, das in Eingriff mit dem Innengewinde der Durchgangslöcher (110b, 12b) bringbar ist, wodurch jedes der Verbindungselemente (132) in das entsprechende Durchgangsloch (110b, 112b) durch Zusammengreifen des Innen- und des Außengewindes einsetzbar ist.

11. Vorrichtung nach einem der vorhergehenden Ansprüche, **dadurch gekennzeichnet, daß** die Verbindungselemente (132) jeweils aufweisen:
- einen Buchsenbereich (232) mit einem Innenvolumen (234), oberen (239) und unteren Abschnitten (231) und wenigstens einem vertikalen Schlitz (233, 241), der in entsprechenden der oberen und unteren Abschnitte ausgebildet ist, wobei wenigstens einer der Schlitze (233) das Innenvolumen (234) erweiterbar und zusammenziehbar macht, wobei der untere Abschnitt (231) eine sich verjüngende Außenseite (235) zum Sitzen auf dem unteren verjüngten Bereich des entsprechenden Durchgangslochs (210, 212) aufweist, derart, daß ein zwangsweise erfolgender Vorschub des Buchsenbereichs (232) in den verjüngten Bereich des Durchgangslochs (210, 212) das Verengen des wenigstens einen Schlitzes (233) bewirkt, wodurch sich das Innenvolumen zusammenzieht, und
- einen Kappenbereich (242) mit einer Öffnung (243) im Boden und einer inneren Kammer, die sich von diesem nach oben erstreckt, zum Verbinden mit und gleitenden Aufnehmen des oberen Abschnitts des Buchsenbereichs (233), und mit einer Außengewinde (247), das in Eingriff mit dem Gewinde des entsprechenden Lochs bringbar ist, derart, daß der Kopfbereich (122) der Knochenschraube vor dem Einsetzen frei drehbar in dem Innenvolumen des Buchsenbereichs (232) des Verbindungselements (132) ist, und wobei das Einsetzen des Schafts (126) der Knochenschraube und des Verbindungselements in das entsprechende Durchgangsloch (210, 212) und das Einsetzen des Schafts (126) der Knochenschraube unter einem gewählten Winkel bewirken, daß die sich verjüngende Außenfläche des Buchsenbereichs (233) am sich verjüngenden Bereich des Durchgangslochs (210, 212) anliegt und zwangsweise gegen diesen vorgeschoben wird, so daß der Kopf (122) der Schraube in dem zusammengezogenen halbkugelförmigen Innenvolumen blockiert ist, und der Gewindevorschub des Kappenbereichs (242) in das Durchgangsloch (210, 212) einen zusätzlichen zwangsweisen Vorschub des Buchsenbereichs (232) gegen den sich verjüngenden Bereich des Durchgangslochs bewirkt.

12. Vorrichtung nach Anspruch 11, **dadurch gekennzeichnet, daß** der Buchsenbereich (232) einen oberen Abschnitt mit im wesentlichen konstantem Durchmesser aufweist, der an einem seiner äußeren Enden eine sich nach außen erstreckende ringförmige Lippe (240) aufweist,
- und **daß** die Öffnung (243) im Boden des Kappenbereichs (242) eine nach innen gerichtete ringförmige Lippe (245) aufweist, und
- wenigstens einer der vertikalen Schlitze (233) im oberen Abschnitt des Buchsenbereichs (232) den oberen Abschnitt desselben aufweitbar und zusammenziehbar macht, so daß der obere Abschnitt des Buchsenbereichs (232) zwangsweise in die Öffnung (243) im Boden des Kappenbereichs (242) eingeführt werden kann, so daß er in der inneren Kammer desselben durch manuelle Presspassung zwischen der einwärts gerichteten ringförmigen Lippe (245) des Kappenbereichs und der nach außen gerichteten ringförmigen Lippe (240) des Buchsenbereichs gehalten ist.

13. Vorrichtung nach Anspruch 11 oder 12, **dadurch gekennzeichnet, daß** die innere Kammer des Kappenbereichs eine sich verjüngende Fläche (244) aufweist, so daß ein Vorschub desselben in das Loch (210, 212) eine nach innen gerichtete, gegen den oberen Abschnitt des Buchsenbereichs (232) wirkende Kraft bewirkt, wodurch der wenigstens eine vertikale Schlitz (241) im oberen Abschnitt verengt wird und der obere Abschnitt sich zusammenzieht und den Kopf (122) der Schraube in dem inneren halbkugelförmigen Volumen des Buchsenbereichs (234) weiter blockiert.

14. Vorrichtung nach einem der vorhergehenden Ansprüche, **dadurch gekennzeichnet, daß**
- die Platte eine langgestreckte Platte (300) mit einem ersten und einem zweiten längsverlaufenden Bereich sowie einer von diesen begrenzten Mitte (307) ist; wobei die Vorrichtung ferner aufweist:
- wenigstens ein langgestrecktes Loch (302, 305), daß sich durch wenigstens entweder den ersten oder den zweiten längsverlaufenden Bereich erstreckt und ein entferntes Ende (312) sowie ein proximales Ende (314) und die Enden verbindende Seitenwände (310a, 310b) aufweist, wobei jede der Seitenwände eine schräge Flächenausbildung aufweist;
- eine Einrichtung in wenigstens dem anderen der ersten oder zweiten längsverlaufenden Bereiche, mittels welcher die Platte an einem ersten der mehreren Knochensegmente befestigbar ist;
- wenigstens ein entsprechendes Verbindungselement (132), das in das wenigstens eine langgestreckte Loch (302, 305) einsetzbar, entlang den schrägen Flächen gleitend verschiebbar und in dem langgestreckten Loch blockierbar ist.

15. Vorrichtung nach Anspruch 14, **dadurch gekennzeichnet, daß** jeder der längsverlaufenden Bereiche der Platte wenigstens ein langgestrecktes Loch (302, 305) aufweist.

16. Vorrichtung nach Anspruch 14 oder 15, **dadurch gekennzeichnet, daß** die Platte ferner wenigstens ein Rundloch (301, 303, 304, 306) und die Einrichtung eine Halteschraube (120) aufweist, die durch das wenigstens eine Rundloch einsetzbar ist, um zusätzliche Fixierungsfestigkeit zu bewirken.

17. Vorrichtung nach einem der Ansprüche 14 bis 16, **dadurch gekennzeichnet, daß** das wenigstens eine langgestreckte Loch (302, 305) sich in Richtung der Längsachse der Platte erstreckt, wobei die Seitenwände (310a, 310b) entlang einer zur Ebene der Platte (300) senkrechten ersten Achse und entlang einer mit dem Loch (302, 305) selbst koaxialen Achse zusammenlaufen, so daß das Loch (302, 305) am Boden schmaler als an der Oberseite ist und der Abstand zwischen den Seitenwänden am proximalen Ende (314) größer als am entfernten Ende (312) ist,
- und das Verbindungselement (132) zylindrisch und zur Bildung eines kegelstumpfförmigen Abschnitts verjüngt ist,
- wobei die äußere Ausbildung ein blockierbares Zusammengreifen mit der Seitenwandmorphologie des entsprechenden langgestreckten Lochs (302, 305) ermöglicht.

18. Vorrichtung nach einem der Ansprüche 14 bis 16, **dadurch gekennzeichnet, daß** das wenigstens eine langgestreckte Loch (302, 305) in Richtung der Längsachse der Platte (300) verläuft, wobei die Seitenwand (310a, 310b) des Lochs wenigstens einen schrägen Kanal (333a, 333b) aufweist, der mit einer ausgenommenen unteren Lippe (335a, 335b), welche die untere Erstreckung des Kanals bildet, die nach unten in die Platte geneigt ist, und einem oberen Lippenbereich (337a, 337b) versehen ist, der die obere Erstreckung des Kanals bildet, wobei das entfernte Ende (328) des Kanals offen ist,
- und mit einem Verbindungselement (350), das einen Ringflansch (360) aufweist, der sich vom Körper des Elements nach außen erstreckt und in Anlage an der unteren Lippe (335a, 335b) des langgestreckten Lochs bringbar und durch fortgesetztes Einführen der Schraube (120) nach unten entlang der Schräge des Kanals antreibbar ist.

## Revendications

1. Dispositif d'implantation orthopédique qui bloque une vis polyaxiale (120) sur une structure d'implant à travers laquelle elle est insérée, comprenant :
une plaque (100) ayant une pluralité de trous traversants (110a, 112a),
une pluralité de vis pour ostéosynthèse (120) ayant chacune une partie de tête (122), la partie de tête étant hémisphérique, et une tige (126), la partie de tige (126) pouvant être insérée à travers un trou traversant correspondant (110, 112) dans la plaque (100a) et dans la structure d'implant, **caractérisé en ce que** le dispositif comprend également :
une pluralité d'éléments de couplage (132), chacun pouvant être inséré dans un trou correspondant des trous traversants (110a, 112a), l'élément de couplage (132) ayant un volume récepteur intérieur (140a) conformé pour retenir la partie de tête hémisphérique et adapté pour que le volume interne (140a) puisse se dilater ou se contracter par l'application d'une force radiale pour recevoir et/ou bloquer la partie de tête (122) de la vis (120) selon l'angle souhaité dans la structure d'implant, de sorte que la partie de tête (122) soit montée à rotation libre dans l'élément de couplage (132) avant insertion, et la tige (126) de la vis pour ostéosynthèse (120) et l'élément de couplage (132) peuvent être insérés dans un trou traversant correspondant (110a, 112a) et la tige (126) de la vis pour ostéosynthèse peut être insérée dans l'os selon un angle choisi dans une plage prédéterminée d'angles comprenant des angles non perpendiculaires par rapport à la plaque.

2. Dispositif selon la revendication 1, **caractérisé en ce que** l'élément de couplage (132) comprend par ailleurs au moins une fente axiale (149) qui permet de dilater le volume intérieur (140a) pour ainsi faciliter l'insertion de la partie de tête (122) de la vis dans celui-ci, et de le contracter lors de l'insertion de l'élément de couplage (132) dans le trou traversant correspondant (110a, 112a), bloquant de la sorte la partie de tête (122) dans le volume intérieur (140a) de l'élément de couplage.

3. Dispositif selon la revendication 2, **caractérisé en ce que** la fente axiale (149) s'étend sur toute la longueur de l'élément de couplage (132).

4. Dispositif selon l'une quelconque des revendications précédentes, **caractérisé en ce que** les éléments de couplage (132) comprennent chacun un volume récepteur intérieur hémisphérique (140a) défini par une surface intérieure incurvée qui forme une douille réceptrice dans laquelle la partie de tête hémisphérique (122) peut être introduite.

5. Dispositif selon l'une quelconque des revendications précédentes, **caractérisé en ce que** l'élément de couplage (132) a une surface externe axialement amincie (134) et le trou traversant correspondant (110a, 112a) dans lequel l'élément de couplage (132) peut être inséré a un amincissement correspondant vers l'intérieur, ce qui permet, lors de l'insertion de l'élément de couplage (132) dans le trou traversant correspondant (110a, 112a), d'amincir la au moins une fente (149), amenant la surface intérieure incurvée de l'élément de couplage (132) à bloquer la partie de tête hémisphérique (122) de la vis (120) selon un angle d'insertion défini.

6. Dispositif selon l'une quelconque des revendications précédentes, **caractérisé en ce que** la partie de tête (122) de chacune des vis (120) comprend un évidement (130) dans lequel un outil de vissage peut être engagé pour insérer la vis (120) à travers le trou correspondant (110, 112) et dans l'os.

7. Dispositif selon l'une quelconque des revendications précédentes, **caractérisé en ce que** chacun des éléments de couplage (132) comprend un évidement de surface supérieur (138a), à travers lequel l'outil de vissage peut être inséré, l'évidement de surface supérieur (138a) étant aligné avec l'évidement (130) de la tête de la vis (120) de sorte qu'un tournevis puisse insérer la vis (120) dans l'os.

8. Dispositif selon l'une quelconque des revendications précédentes, **caractérisé en ce qu'**au moins l'un de la pluralité de trous traversants (110a, 112a) comprend par ailleurs un évidement annulaire (182) formé dans une partie de paroi latérale supérieure (115) du trou, et un anneau d'encliquetage (180) situé dans l'évidement (182), l'anneau d'encliquetage (180) déviant vers l'intérieur au cours de l'insertion de l'élément de couplage (132) pour permettre à l'élément de couplage de se déplacer dans le trou traversant (110a, 112a), et l'anneau d'encliquetage (180) se ré-encliquetant dans un état non dévié une fois que l'élément de couplage (132) s'est complètement logé dans le trou traversant (110a, 112a), empêchant de la sorte l'élément de couplage (132) de ressortir du trou traversant (110a, 112a).

9. Dispositif selon l'une quelconque des revendications précédentes, **caractérisé en ce que** la plaque (100a) comprend une première extrémité et une seconde extrémité, un premier groupe (110a) des trous traversants étant disposé à la première extrémité et un second troupe (112a) des trous traversants étant disposé à la seconde extrémité.

10. Dispositif selon l'une quelconque des revendications précédentes, **caractérisé en ce que** les trous traversants (110b, 112b) comprennent un filet de surface intérieur et chacun des éléments de couplage (132) comprend un filet de surface extérieur qui peut s'engager sur le filet de surface intérieur des trous traversants (110b, 112b), de sorte que chacun des éléments de couplage (132) puisse s'insérer dans le trou traversant correspondant (110b, 112b) par engagement des filets de surface intérieur et extérieur.

11. Dispositif selon l'une quelconque des revendications précédentes, **caractérisé en ce que** les éléments de couplage (132) comprennent chacun :
un partie de douille (232) ayant un volume intérieur (234), des sections supérieure (239) et inférieure (231), et au moins une fente verticale (233, 241) formée sur les sections correspondantes des sections supérieure et inférieure, au moins l'une des fentes (233) rendant le volume intérieur (234) dilatable et contractable, la section inférieure (231) ayant une surface extérieure amincie (235) pour s'appuyer contre la partie amincie inférieure du trou traversant correspondant (210, 212) de sorte qu'un avancement à force de la partie de douille (232) dans la partie amincie du trou traversant (210, 212) amène au moins l'une des fentes (233) à s'amincir de sorte que le volume intérieur se contracte, et
une partie de chapeau (242) ayant une ouverture (243) dans sa partie inférieure et une chambre intérieure s'étendant vers le haut depuis celle-ci pour joindre, et y retenir à glissement, la section supérieure de la partie de douille (233), et un filet extérieur (247) qui peut s'engager sur le filet du trou correspondant,
de sorte que la partie de tête (122) de la vis pour ostéosynthèse soit montée à rotation libre dans le volume intérieur de la partie de douille (232) de l'élément de couplage (132) avant insertion, et que l'insertion de la tige (126) de la vis pour ostéosynthèse et de l'élément de couplage dans le trou traversant correspondant (210, 212) et l'insertion de la tige (126) de la vis pour ostéosynthèse selon un angle sélectionné amènent la surface externe amincie de la partie de douille (233) à se loger et à avancer à force contre la partie amincie du trou traversant (210, 212) de sorte que la tête (122) de la vis soit bloquée dans le volume intérieur hémisphérique contracté, un avancement par vissage de la partie de chapeau (242) dans le trou traversant (210, 212) provoquant un avancement supplémentaire à force de la partie de douille (232) contre la partie amincie du trou traversant.

12. Dispositif selon la revendication 11, **caractérisé en ce que** la partie de douille (232) comprend une section supérieure de diamètre sensiblement constant ayant une lèvre annulaire (240) s'étendant vers l'extérieur à son extrémité extrême,
et **en ce que** l'ouverture (243) ménagée dans le fond de la partie de chapeau (242) comprend une lèvre annulaire (245) dirigée vers l'intérieur, et
au moins l'une des fentes verticales (233) de la section supérieure de la partie de douille (232) rend sa section supérieure susceptible de se dilater et de se contracter de sorte que la section supérieure de la partie de douille (232) puisse être insérée à force dans l'ouverture (243) du fond de la partie de chapeau (242) de sorte qu'elle puisse y être retenue dans la chambre intérieure par engagement par interférence manuelle de la lèvre annulaire (245), dirigée vers l'intérieur, de la partie de chapeau et de la lèvre annulaire (240), s'étendant vers l'extérieur, de la partie de douille.

13. Dispositif selon la revendication 11 ou 12, **caractérisé en ce que** la chambre intérieure de la partie de chapeau comprend une surface amincie (244) telle que son avancement dans le trou (210, 212) provoque une force dirigée vers l'intérieur contre la section supérieure de la partie de douille (232), faisant en sorte que la au moins une des fentes verticales (241) de la section supérieure s'amincisse et amène la section supérieure à se contracter et à encore bloquer la tête (122) de la vis dans le volume hémisphérique intérieur de la partie de douille (234).

14. Dispositif selon l'une quelconque des revendications précédentes, **caractérisé en ce que** :
ladite plaque est une plaque allongée (300) comprenant une première et une seconde parties longitudinales et une partie centrale (307) définie entre elles, ledit dispositif comprenant par ailleurs :
au moins un trou allongé (302, 305) s'étendant à travers au moins l'une des première et seconde parties longitudinales, ayant une extrémité éloignée (312) et une extrémité proche (314) et lesdites parois latérales (310a, 312a) raccordant les extrémités, chacune des parois latérales ayant une conformation de surface inclinée;
un moyen dans au moins l'autre des première et seconde parties longitudinales par lequel la plaque peut être fixée à un premier de la pluralité de segments d'os;
au moins un élément de couplage correspondant (132) pouvant être inséré dans le au moins un trou allongé (302, 305), pouvant glisser le long des conformations de surface inclinées et pouvant se bloquer dans le trou allongé.

15. Dispositif selon la revendication 14, **caractérisé en ce que** chacune des parties longitudinales de la plaque comprend au moins un trou allongé (302, 305).

16. Dispositif selon la revendication 14 ou 15, **caractérisé en ce que** la plaque comprend par ailleurs au moins un trou rond (301, 303, 304, 306) et le moyen comprend une vis de retenue (120) qui peut être insérée à travers le au moins un trou rond pour y établir une fixation de solidité accrue.

17. Dispositif selon l'une quelconque des revendications 14 à 16, **caractérisé en ce que** le au moins un trou allongé (302, 305) s'étend dans la direction de l'axe allongé de la plaque, les parois latérales (310a, 310b) étant amincies sur un premier axe perpendiculaire au plan de la plaque (300) et, sur un second axe, co-axial avec le trou (302, 305) lui-même de sorte que le trou (302, 305) soit plus étroit dans la partie inférieure que dans la partie supérieure et que la séparation des parois latérales (310a, 310b) soit plus large à l'extrémité proche (314) qu'à l'extrémité éloignée (312),
et l'élément de couplage (132) est cylindrique et aminci pour former une section tronconique,
la conformation extérieure permettant un engagement bloquant avec la morphologie de la paroi latérale du trou allongé correspondant (302, 305).

18. Dispositif selon l'une quelconque des revendications 14 à 16, **caractérisé en ce que** le au moins un trou allongé (302, 305) s'étend dans la direction de l'axe allongé de la plaque (300), la paroi latérale (310a, 310b) du trou comprenant au moins un canal incliné (333a, 333b), le canal comprenant une lèvre inférieure évidée (335a, 335b) qui forme la dimension inférieure du canal qui est incliné vers le bas dans la plaque et une partie de lèvre supérieure (337a, 337b) qui définit la dimension supérieure du canal, l'extrémité éloignée (328) du canal étant ouverte,
et un élément de couplage (350) qui comprend une bride annulaire (360) s'étendant vers l'extérieur depuis le corps de l'élément et qui peut se loger sur la lèvre inférieure (335a, 335b) du trou allongé et peut être entraînée en continuant l'insertion de la vis (120) vers le bas sur la pente du canal.
